# EUROPEAN PATENT SPECIFICATION

(11) **EP 3 877 012 B1**
(45) Date of publication and mention of the grant of the patent: **25.09.2024**
(21) Application number: 19881968.2
(22) Date of filing: 05.11.2019
(51) Int. Cl.: A61K 9/70, A61P 31/04, A61P 31/18, A61K 9/51, A61K 38/00, C07K 14/405, A61K 35/747, A61K 31/675, A61P 31/12

(54) **METHODS AND COMPOSITION FOR TREATING MICROBIAL INFECTIONS**
VERFAHREN UND ZUSAMMENSETZUNGEN ZUR BEHANDLUNG VON MIKROBIELLEN INFEKTIONEN
MÉTHODES ET COMPOSITION POUR LE TRAITEMENT D'INFECTIONS MICROBIENNES

(30) Priority: 05.11.2018 US 201862756041 P; 15.07.2019 US 201962874001 P; 18.07.2019 US 201962875923 P; 01.08.2019 US 201962881633 P
(43) Date of publication of application: 15.09.2021
(73) Proprietor: University of Louisville Research Foundation, Inc., Louisville, KY 40202 (US)
(72) Inventor: STEINBACH-RANKINS, Jill, Louisville, Kentucky 40202 (US); PALMER, Kenneth, Louisville, Kentucky 40202 (US)
(74) Representative: Fish & Richardson P.C.
(86) International application number: PCT/US2019/059842
(87) International publication number: WO 2020/097062

(56) References cited:
- WO-A1-2011/045764
- WO-A1-2011/045764
- WO-A1-2016/125173
- WO-A1-2016/132370
- CN-A- 108 589 045
- CN-A- 109 288 819
- US-A1- 2009 061 496
- US-A1- 2016 361 382
- US-A1- 2018 044 818
- BRESHEARS L.M. ET AL.: "Lactobacillus crispatus inhibits growth of Gardnerella vaginalis and Neisseria gonorrhoeae on a porcine vaginal mucosa model", BMC MICROBIOLOGY, vol. 15, no. 1, 1 December 2015 (2015-12-01), XP055929614, Retrieved from the Internet <URL:https://bmcmicrobiol.biomedcentral.com/track/pdf/10.1186/s12866-015-0608-0.pdf> DOI: 10.1186/s12866-015-0608-0
- SIENIAWSKA ET AL.: "Natural Terpenes Influence the Activity of Antibiotics against Isolated Mycobacterium tuberculosis", MED PRINC PRACT 2017, vol. 26, 23 November 2016 (2016-11-23), pages 108 - 112, XP055706054

## Description

### TECHNICAL FIELD

This disclosure generally relates to methods and composition for treating microbial infections.

### BACKGROUND

Despite decades of research, sexually transmitted infections (STIs) such as human immunodeficiency virus (HIV-1) and herpes simplex virus type 2 (HSV-2), remain incurable. As such, reducing new cases of these STIs, especially in high-risk female populations, has become a major focus of global health initiatives. In addition, bacterial vaginosis (BV) is a significant condition resulting from the disruption of the diverse microbial communities that maintain host homeostasis and innate defense in the female reproductive tract (FRT). Similar to viral STIs, BV disproportionately affects disadvantaged and minority populations, and contributes to persistent disparities in women's health and social outcomes.

Improved compositions and methods for treating microbial infections in the female reproductive tract would be beneficial.

WO 2011/045764 describes a product that includes a mesh of soluble nanofibers encapsulating viable bacteria cells capable of producing a peptide having antimicrobial properties.

WO 2016/132370 describes starch-based fibers, compositions including the same, a method of preparing said starch-based fibers, and kits and methods for use of said starch-based fibers including but not limited to oral delivery of cells (e.g., probiotic microorganisms) and/or molecules of interest (e.g., nutrients).

US 2009/061496 describes a method of preserving organisms in viable form. The method includes suspending organisms in a solution of electrospinnable polymer, drawing droplets of said solution through a spinneret, and applying an electrostatic field to said droplets under electrospinning conditions so as to form fibers having a diameter no greater than about 5 µm within which distinct organisms are encapsulated in viable form.

CN 109288819 describes a segmented intestine targeted nano fibrous membrane, preparation method, and application containing Quercetin and a prebiotic factor. The method includes preparing the chitin nanometer containing Quercetin using ionic crosslinking benefit, dissolving sodium alginate, polyethylene glycol oxide and poloxamer F127 in distilled water, obtaining a shell spinning solution, and mixing the chitin nanometer solution containing Quercetin and the prebiotic factor with poly-vinyl alcohol solution, obtaining a stratum nuclear spinning solution. The method includes coaxial electrostatic spinning carried out at room temperature, obtaining the nano fibrous membrane containing Quercetin and the prebiotic factor.

CN 108589045 describes nano fibrous membrane and preparation and application loading prebiotics and/or probiotics. Polyvinyl alcohol is dissolved in aseptic deionized water and a polyvinyl alcohol spinning solution is obtained. Prebiotics are added into a polyvinyl alcohol spinning solution again and stirred evenly to obtain a polyvinyl alcohol/prebiotics spinning solution. Probiotics bacterial liquid is added in polyvinyl alcohol spinning solution or polyvinyl alcohol/prebiotics spinning solution, is stirred evenly, and a polyvinyl alcohol/probiotics spinning solution or polyvinyl alcohol/prebiotics/probiotics spinning solution are obtained. The prebiotics and/or the nano fibrous membrane of probiotics are prepared by electrostatic spinning in gained spinning solution.

### SUMMARY

Antimicrobial (e.g., antibacterial, antiviral) compositions and methods of using such compositions are described herein. The subject matter for which protection is sought is defined in the claims.

Claim 1 describes antimicrobial compositions that includes electrospun fibers including a probiotic embedded therein are provided. The probiotics include *L*. *acidophilus, L. crispatus, L. rhamnosus, L. reuteri, L. jensenii,* and *L. gasseri.* An antimicrobial composition as described herein includes a prebiotic. The prebiotics include fructans (e.g., fructooligosaccharides (FOS), inulins), galactans (galactooligosaccharides (GOS)), pectin, beta-glucans, and xylooligosaccharides.

In some embodiments, the electrospun fiber material is biodegradable or non-biodegradable electrospun fiber material. Representative biodegradable fiber materials include, without limitation, PLGA, mPEG-PLGA, PVA, PEO, PVP, and combinations thereof. A representative non-biodegradable electrospun fiber material includes, without limitation, polycaprolactone (PCL).

Claim 6 describes a method of making an antimicrobial composition. The method includes electrospinning fiber material and a probiotic into electrospun fibers that include the probiotic embedded therein, thereby making an antimicrobial composition, wherein the probiotic is a *Lactobacillus* spp. selected from the group consisting of *L. crispatus, L. rhamnosus, L. reuteri, L. jensenii,* and *L. gasseri,* and wherein the antimicrobial composition further comprises a prebiotic, wherein the prebiotic is selected from the group consisting of fructans (e.g., fructooligosaccharides (FOS), inulins), galactans (galactooligosaccharides (GOS)), pectin, beta-glucans, and xylooligosaccharides.

In some embodiments, the implanting step provides a sustained release of the probiotic into the reproductive tract of the female.

In one aspect, antiviral compositions including electrospun fibers including griffithsin-encapsulated nanoparticles embedded therein are provided. In another aspect, antiviral compositions including electrospun fibers including griffithsin embedded therein are provided.

Representative electrospun fiber material includes, without limitation, PLGA, mPEG-PLGA, PVA, PEO, PVP, polycaprolactone (PCL), and combinations thereof. In some embodiments, the electrospun fibers are hydrophobic, hydrophilic, or a combination thereof.

In some embodiments, the griffithsin is a modified griffithsin. A representative modified griffithsin includes a Met78Gln variation and results in an oxidation-resistant griffithsin.

In some embodiments, the composisition further includes an active agent. Representative active agents include, without limitation, a non-griffithsin anti-retroviral (e.g., tenofovir, tenofovir disoproxil fumarate, emtricitabine, tenofovir alafenamide, dapivirine, elvitegravir, raltegravir, cabotegravir), antiviral against HSV-2 or HIV-1 (e.g., acyclovir or HIV-1 non-ARV), proteins (e.g., cyanovirin) / antibodies, oligonucleotides, cytokines, peptides, antimicrobials (e.g., metronidazole, clindamycin, tinidazole).

In yet another aspect, antiviral compositions are provided that are made by a process including providing griffithsin-encapsulated nanoparticles; and electrospinning fiber material and the griffithsin-encapsulated nanoparticles into electrospun fibers including the griffithsin-encapsulated nanoparticles embedded therein.

In still another aspect, methods of treating a viral infection in the reproductive tract of a female are provided. Such methods typically include implanting an antiviral composition as described herein into the reproductive tract of the female. In some embodiments, the implanting step provides a sustained release of the griffithsin into the reproductive tract of the female. In some embodiments, the sustained release is about 90 days. In some embodiments, the implanting step provides a rapid-release of the griffithsin into the reproductive tract of the female.

Representative viral infections are caused by a virus such as, without limitation, HIV-1, HIV-2, HSV-1, HSV-2, Hepatitis C (HCV), and severe acute respiratory syndrome coronavirus (SARS-CoV).

Unless otherwise defined, all technical and scientific terms used herein have the same meaning as commonly understood by one of ordinary skill in the art to which the methods and compositions of matter belong. Although methods and materials similar or equivalent to those described herein can be used in the practice or testing of the methods and compositions of matter, suitable methods and materials are described below. In addition, the materials, methods, and examples are illustrative only and not intended to be limiting. All publications, patent applications, patents, and other references mentioned herein are incorporated by reference in their entirety.

### DESCRIPTION OF DRAWINGS

*Part A-Methods and Compositions for Treating Bacterial Infections*

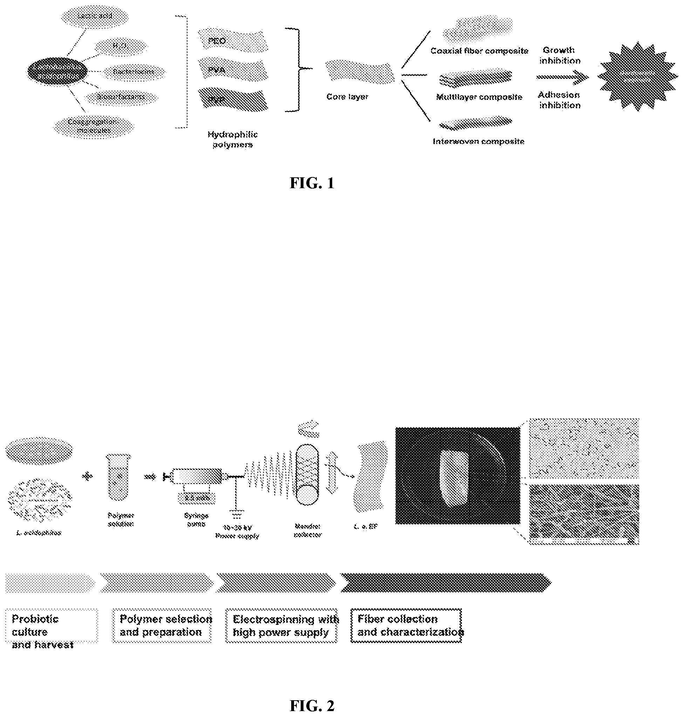

   FIG. 1. A sketch of pre- and probiotic delivery platforms to prevent and treat the BV.
   FIG. 2. An example fabrication process of a probiotic electrospun fiber.

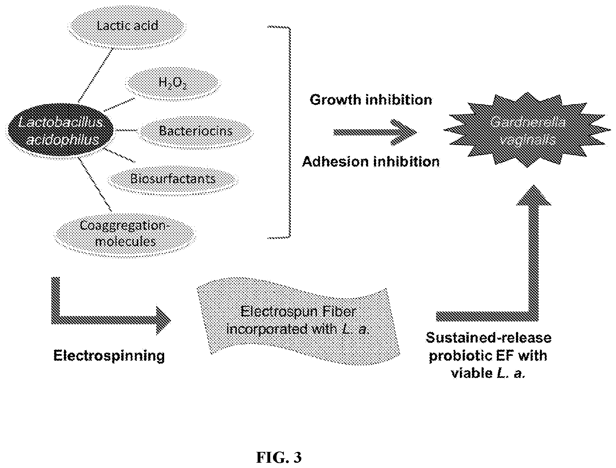

   FIG. 3. A schematic showing exemplary methods that can be used to construct an electrospun fiber that includes a probiotic.
   FIG. 4. One exemplary method used to construct an electrospun fiber that includes a probiotic.
   FIG. 5. SEM images of 5% PEO electrospun fibers containing different concentrations of L. acidophilus. (5A) Blank PEO fiber and PEO fibers loaded with: (5B) 5 × 10e6, (5C) 5 × 10e6 CFU L. acidophilus per mg fiber (CFU/mg); 5 × 10e8 CFU L. acidophilus per mg fiber (5D); the corresponding sizes of encapsulated *L. acidophilus* (5E).
   FIG. 6. SEM images of 5% PEO electrospun fibers containing *L. acidophilus* in different formulations. (6A) PEO La fiber spun in water; (6B) PEO La fiber spun in MRS broth; (6C) PEO La fiber in MRS with 5% glycerol; (6D) PEO with lyophilized La spun in MRS; (6E) PEO with lyophilized La spun in MRS containing 5% glycerol.

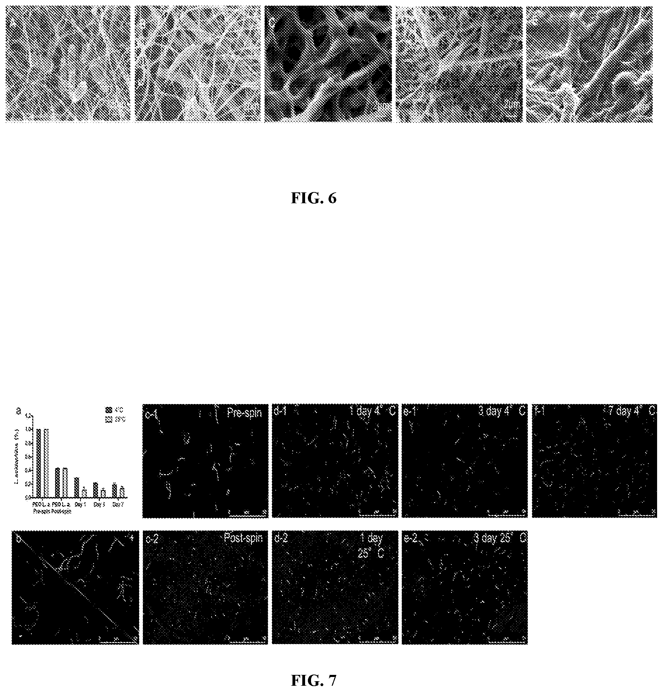

   FIG. 7. Probiotic L. acidophilus viability and stability in electrospun PEO fibers at different time points. (7A) Overall La viability before and after electrospinning at room temperature (25°C) and refrigerated conditions (4°C) using BacLight live/dead assay. Images of BacLight stained La were taken at different time points after fiber synthesis and storage: (7B) Positive and negative controls; (7C-1) PEO La solution prior to electrospinning; (7C-2) PEO La fiber dissolved post-electrospinning; and after (7D-1) 1 day storage at 4°C; (7D-2) 1 day at 25°C; (7E-1) 3 days at 4°C; (7E-2) 3 days at 25°C; (7F-1) 7 days at 4°C; and (7F-2) 7 days at 25°C.
   FIG. 8. The viability and stability of probiotic L. acidophilus in different electrospun PEO and PVA fiber formulations at different time points. Three storage conditions were tested, 25°C, 4°C, and -20°C, for PEO (left) and PVA (right) EFs. (8A) L. a. EFs spun in water, (8B) L. a. EFs spun in MRS, (8C) L. a. EFs spun in MRS broth with 5% glycerol.
   FIG. 9. La viability from 5% PEO (9A, 9B) and 10% PVA (9C, 9D) fibers determined using CFU counts. Probiotic viability prior to and immediately post-electrospinning (9A, 9C) and after storage of up to 1 week (9B, 9D). Over 5 × 10e6 CFU/mg were recovered from the EFs.
   FIG. 10. A comparison of La viability between PEO and PVA fibers at 4°C. High variability was maintained at 4C, and no statistically significant differences were observed between PEO and PVA La fibers (p<0.05).
   FIG. 11. Free La and Gv co-culture. A significant decrease in clinically isolated Gv viability was seen after 4 hr co-incubation of La-Gv co-culture.
   FIG. 12. La viability in different (12A) PEO and (12B) PVA formulations after electrospinning as a function of CFU per mg fiber. The amount of bacteria loaded in polymer solution prior to electrospinning, determined via CFU counts, was considered 100% viable.
   FIG. 13. Free La and Gv co-culture. (13A) Free La and Gv were co-cultured in NYC II broth at equal concentrations, or (13B) with La one log lower than Gv Free La demonstrated inhibition of GV growth 4 and 24 hours after incubation.
   FIG. 14. La PEO EF and Gv co-culture. (14A) La EF and Gv were co-cultured in NYC III broth at equal concentration, or (14B) with La one log lower than Gv La EFs demonstrated inhibition of Gv growth 4 and 24 hr after incubation.
   FIG. 15. (15A) Pictures of the electrospinner used to fabricate polymer fibers from a polymer solution formulated prior to spinning. (15B) A fiber formed via electrospinning, composed of PEO, probiotic, and prebiotic.
   FIG. 16. (16A) Experimental design schematic of probiotic (La or Lcr), prebiotic, and Gv combinations for bacterial cell co-culture experiments. Each tube was serially diluted and plated after (16B) 4 and (16C) 24 hours growth.
   FIG. 17. Representative SEM images of (Left) Lcr and (Right) La fibers containing prebiotic (here, inulin). Scale bars represent 2 um.
   FIG. 18. Viability of La probiotics in fibers stored in room temperature conditions for 60 days.
   FIG. 19. Viability of free Lcr and Gv, cultured alone with different prebiotics.
   FIG. 20. Viability of free La and Gv, cultured alone with different prebiotics.
   FIG. 21. Viability of free Lcr and Gv, in co-culture conditions with different prebiotics.
   FIG. 22. Viability of free La and Gv, in co-culture conditions with different prebiotics.
*Part B-Methods and Compositions for Treating Viral Infections*

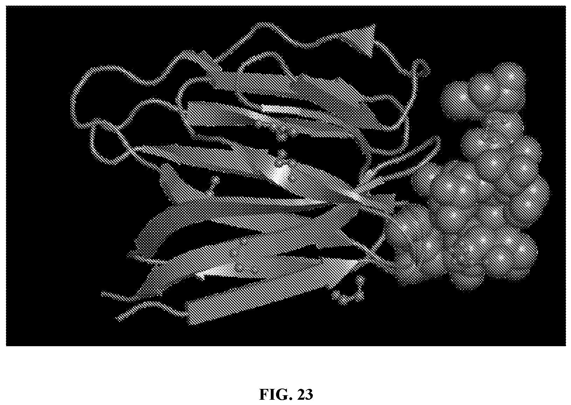

   FIG 23. 3D ribbon version of Griffithsin.
   FIG. 24. (24A) Schematic depicting the incorporation of NPs within hydrophilic fibers to create multilayered NP-EF composites. Multilayered fibers consist of an inner layer of hydrophilic polymer (here, PEO) that incorporates GRFT or C6 NPs, and outer hydrophobic layers of PCL to tune GRFT release from the incorporated NPs. (24B) Schematic of NP-EF composites fabricated with varying outer layer thicknesses to modulate GRFT release. The multilayered fiber acts as a NP reservoir, providing localized GRFT release from the composite.
   FIG. 25. Scanning electron microscopy images of (25A) blank, (25B) 50, (25C) 100, and (25D) 200 µg/mg GRFT PLGA NPs, and (25E) blank, (25F) 50, (25G) 100, and (25H) 200 µg/mg GRFT mPEG-PLGA NPs. Scale bars represent 100 nm.
   FIG. 26. The cumulative release of GRFT from mPEG-PLGA and PLGA NPs shown as (26A) total GRFT release or (26B) the percent of total loading. Increased amounts of GRFT were released from mPEG-PLGA NPs relative to PLGA NPs, and total release corresponded with GRFT loading for each formulation. Release values are shown as the mean ± standard deviation of three independent NP batches.
   FIG. 27. Scanning electron microscopy images of GRFT NP-EF composites taken from (27A) inner layer PEO fibers (5% w/w) that incorporate 20% w/w blank mPEG-PLGA NPs, (27B) outer layer PCL fibers, and (27C) a cross-sectional image of an NP-EF composite. Scale bars for panels 27A and 27B represent 2 µm, while the scale bar in panel 27C represents 20 µm.

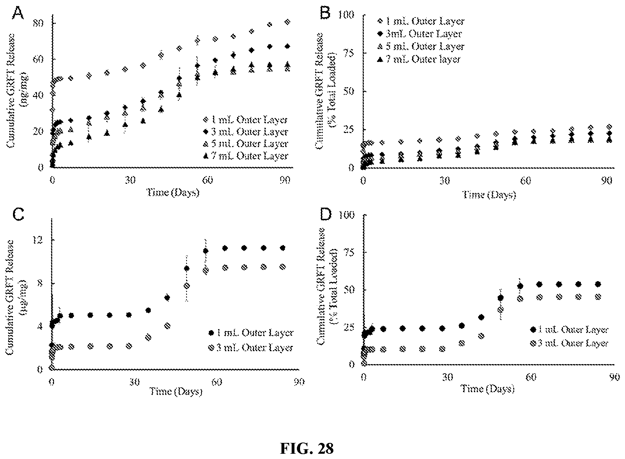

   FIG. 28. The cumulative release of GRFT from multilayered mPEG-PLGA NP-EF composites formulated with 1% w/w NP loading, with different outer layer thicknesses shown as (28A) total GRFT release or (28B) percent of total loading. Subsequent release studies evaluated the cumulative release of GRFT from mPEG-PLGA NP-EF composites formulated with 20% w/w NPs shown as (28C) total GRFT release or (28D) percent of total loading. Overall, GRFT release increased from composites with thinner shells. Griffithsin release values are shown as the mean ± standard deviation of three independent NP-EF batches.

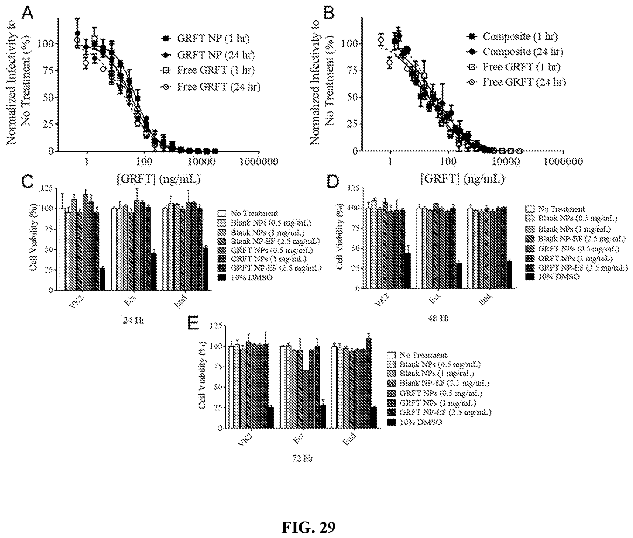

   FIG. 29. Results from *in vitro* HIV-1 inhibition and MTT toxicity assays. Three independent batches of GRFT NPs and NP-EF composites were assessed for their ability to inhibit HIV-1 infection. Nanoparticles or composite eluates were incubated with cells (29A) 1 hr and (29B) 24 hr prior to HIV-1 infection. GRFT released from both platforms exhibited complete HIV-1 inhibition at both 1 and 24 hr, relative to free GRFT. The cytotoxicity of the maximum dose of GRFT NPs and NP-EFs used in the *in vitro* assays was administered to vaginal VK2/E6E7, Ect1/E6E7, and End1/E6E7 cells for (29C) 24, (29D) 48, and (29E) 72 hr was assessed using the MTT assay. Greater than 94% viability was observed in all cell lines for all NPs and NP-EF formulations.

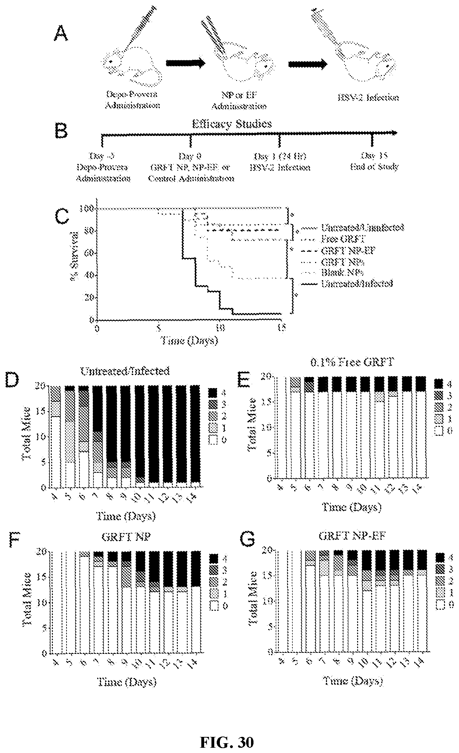

   FIG. 30. Schematic, timetable, and results of *in vivo* HSV-2 efficacy study. (30A) Sequence of murine treatments and (30B) administration schedule from HSV-2 efficacy study. (30C) Kaplan-Meier survival curves from HSV-2 *in vivo* efficacy study. GRFT platforms were assessed for their ability to protect mice against a lethal challenge (LD₉₀) of HSV-2 infection (n=20). Both GRFT NPs and NP-EF composites demonstrated comparable protection (70% and 80%, respectively) relative to free GRFT (85%), and demonstrated a significant increase in protection relative to untreated/infected controls (5% survival). Additionally, blank NPs were found to decrease viral infection and protect 35% mice after 14 d, *p < 0.05. Furthermore, mice were evaluated and scored for progression of infection once daily for 14 d post-infection. Infected mice administered (30D) no treatment, (30E) free GRFT, (30F) GRFT NPs or (30G) GRFT NP-EFs exhibited decreased severity of infection and more gradual progression of infection, relative to untreated/infected animals.

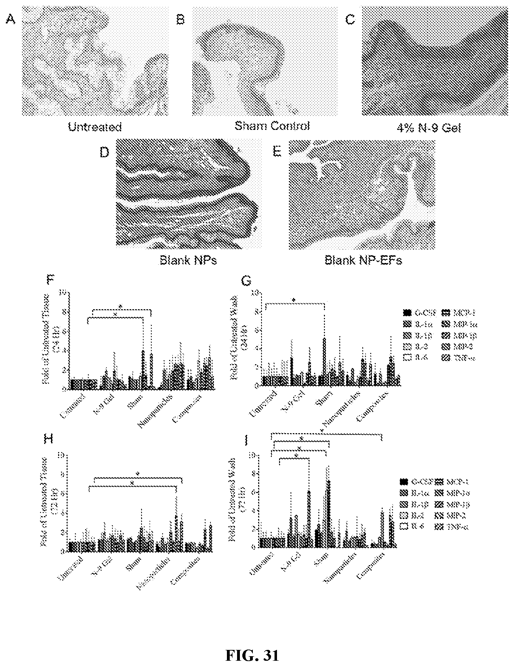

   FIG. 31. The safety of NPs and NP-EF composites was assessed by administering platforms to murine reproductive tracts for 24 and 72 hr. H&E stained tissues of murine reproductive tracts exposed to (31A) no treatment, (31B) sham-treatment, (31C) blank NPs, and (31D) blank NP-EF composites for 24 hr. No indication of tissue inflammation or epithelial disruption was observed after NP or NP-EF administration, relative to untreated controls. Additionally, cytokine expression was evaluated from extracted murine tissue and vaginal lavages collected (31E, 31F) 24 and (31G, 31H) 72 hr after vehicle administration. Cytokine expression was assessed using previously published criteria, with acceptable safety values within a 2 to 5-fold range of expression in the untreated control group. Statistical analysis was used in addition to evaluate possible increased cytokine expression in both tissue and vaginal lavage samples. Mice treated with blank NPs or NP-EF composites expressed similar cytokine levels relative to untreated controls, with no cytokine levels demonstrating over 5-fold increase relative to untreated controls, indicating that the presence of either platform does not induce inflammation or immune response. Statistical significance between untreated controls and other groups represented by * (p < 0.05).
   FIG. 32. Characterization of the average diameter of blank and GRFT NPs. NP diameters were determined using SEM and ImageJ software. Statistical significance between groups represented by *(p < 0.05).
   FIG. 33. The cumulative release of free GRFT from multilayered fibers only, with different outer layer thicknesses shown as (33A) total GRFT release or (33B) percent of total loading. Increased GRFT release was observed from multilayers with thinner shells, although complete release was observed in all samples after 72 hr. Griffithsin release values are shown as the mean ± standard deviation of three independent NP-EF batches.
   FIG. 34. Scanning electron microscopy (SEM) images of (34A-34C) blank, (34D-34F) 1% w/w GRFT, and (34G-34I) 10% w/w GRFT fibers. (34A) 5% PEO, (34B) 20% PVA, (34C) 20% PVP blank fibers; (34D) 5% PEO, (34E) 20% PVA, (34F) 20% PVP fibers incorporating 1% w/w GRFT; and (34G) 5% PEO, (34H) 20% PVA, (34I) 20% PVP fibers incorporating 10% w/w GRFT. Scale bars represent 2 µm.
   FIG. 35. GRFT loading in different hydrophilic fiber formulations. Eluates from GRFT fibers dissolved in PBS were used to determine GRFT loading via ELISA. GRFT loading is expressed as the mean ± standard deviation of triplicate readings of three independent fiber batches. Statistical significance between fiber formulations with the same loading are shown (*p < 0.05).

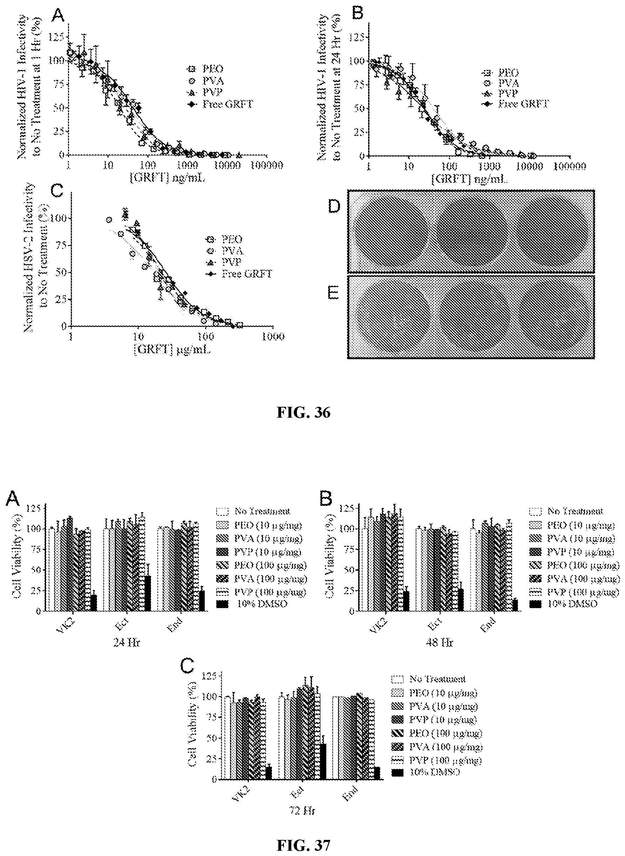

   FIG. 36. GRFT fibers demonstrate complete protection against *in vitro* HIV-1 and HSV-2 infections. Three independent batches of 1% w/w GRFT fibers were assessed for their ability to inhibit HIV-1 infection. Fiber eluates were incubated with cells (36A) 1 hr and (36B) 24 hr prior to HIV-1 infection. GRFT released from all three fiber formulations achieved complete efficacy against HIV-1 infection, similar to free GRFT. (36C) *In vitro* HSV-2 plaque assays were performed using 10% w/w GRFT fibers, which similarly achieved complete efficacy against HSV-2 infection *in vitro,* similar to free GRFT. (36D) Wells treated with GRFT fibers showed decreased plaque sizes and numbers relative to (36E) untreated (or blank fiber-treated) cells infected with HSV-2. The percent infection relative to untreated/infected control groups for both HIV-1 and HSV-2 assays is shown as the mean ± standard deviation of triplicate readings.
   FIG. 37. The cytotoxicity of PEO, PVA, and PVP fibers administered to vaginal VK2/E6E7, Ect1/E6E7, and End1/E6E7 cells for (37A) 24, (37B) 48, and (37C) 72 hr was assessed using the MTT assay. Greater than 93% viability was observed across all cell lines for all fiber formulations.

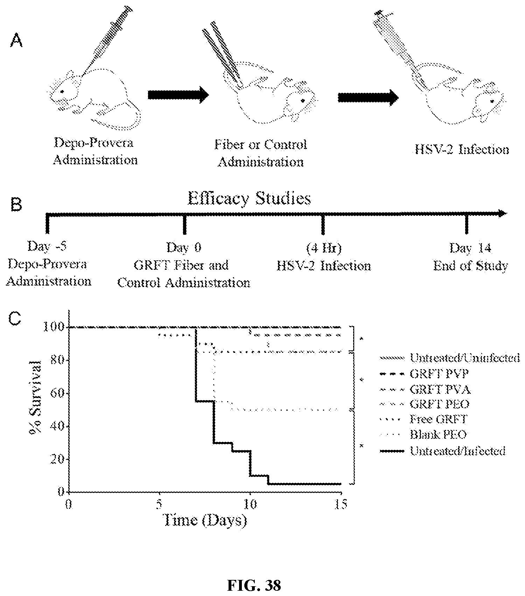

   FIG. 38. Schematic timetable and Kaplan-Meier survival curves of *in vivo* HSV-2 efficacy study. (38A) Sequence of murine treatments during the course of the efficacy study and (38B) timeline of HSV-2 efficacy study. (38C) GRFT fibers (10% w/w) were assessed for their ability to protect mice against a lethal challenge (LD₉₀) of HSV-2 infection (n=20). All GRFT fiber formulations demonstrated strong protection against HSV-2 infection, resulting in 85 to 100% murine survivability. Blank fibers also demonstrated partial protection and showed significant survivability (50%), relative to the untreated/infected control group. In contrast, untreated/infected mice demonstrated only 5% survivability. Statistical significance between experimental groups is shown as (*p < 0.05).
   FIG. 39. Griffithsin fibers protect mice against HSV-2 infection. Mice were administered 10% w/w GRFT fibers 4 hr prior to HSV-2 infection (LD₉₀). Mice were evaluated and scored for progression of infection once daily for 14 d post-infection. Infected mice were administered (39A) PEO, (39B) PVA, (39C) PVP GRFT fibers, as well as (39D) free GRFT, or (39E) no treatment. Mice administered GRFT fibers and free GRFT exhibited decreased severity of infection, as well as more gradual progression of infection, relative to untreated/infected animals.
   FIG. 40. The *in vivo* safety of rapid-release fibers was assessed by intravaginally administering fibers for 24 and 72 hr. Images depict H&E stained tissues of murine reproductive tracts exposed (40A) no treatment, (40B) N-9 gel, (40C) and sham-treatment, as well as blank (40D) PEO, (40E) PVA, and (40F) PVP fibers for 24 hr. There was no indication of tissue inflammation or epithelial disruption from fiber administration, relative to untreated controls. Similar trends were observed for tissue specimens assessed 72 hr post-administration.
   FIG. 41. Cytokine expression from extracted murine tissue and vaginal lavages collected (41A, 41B) 24 and (41C, 41D) 72 hr after fiber administration. Cytokine expression was assessed concurrently with histology to determine the preliminary safety of rapid-release fibers in a murine model. Mice treated with blank fibers expressed similar cytokine levels relative to untreated controls, indicating that the presence of fibers does not induce inflammation or an inflammatory response (*p < 0.05).
   FIG. 42. Images of H&E stained tissue outliers. (42A) A blank PVA fiber-treated tissue sample replicate demonstrated increased levels of mucin secretion and neutrophil presence; however, no disruption of the vaginal or cervical epithelium was observed. (42B) In contrast, a replicate of N-9 treated tissue exhibited inflammatory markers indicated by the presence of peripheral blood mononuclear cells, goblet cell fusions, and epithelial disruption.
   FIG. 43 shows scanning electron microscopy (SEM) images of PLGA NPs loaded with 10% Q-GRFT (43A), TFV (43B), RAL (43C) and DAP (43D). Scale bar: 200 nm.
   FIG. 44 is a graph showing the in vitro release of the active agents (Q-GRFT, TFV, RAL or DAP) from the PLGA NPs in simulated vaginal fluid (SVF).
   FIG. 45A - 45B are graphs showing the IC50 for each active agent (free) (45A) or encapsulated in a NP (45B).
   FIG. 46A - 46C are graphs showing synergistic interactions between Q-GRFT and free ARVs. IC50 curves are shown following administration of free Q-GRFT in combination with free TFV (46A), free RAL (46B), or free DAP (46C).
   FIG. 47A - 47C are graphs showing synergistic interactions between Q-GRFT NPs and free ARVs. IC50 curves are shown following administration of Q-GRFT NPs with free TFV (47A), free RAL (47B), or free DAP (47C).
   FIG. 48A - 48C are graphs showing synergistic interactions between Q-GRFT NPs and encapsulated ARVs. IC50 curves are shown following administration of Q-GRFT NPs with encapsulated TFV (48A), encapsulated RAL (48B), or encapsulated DAP (48C).
   FIG. 49A - 49B are graphs showing the *in vitro* cytotoxicity of NPs encapsulating different active agents administered to End1/E6E7 (49A) or Ect1/E6E7 (49B) cell lines.
   FIG 50A - 50B are graphs showing the release of Q-GRFT from NPs (50A) or NP-EF composites (50B).

Like reference symbols in the various drawings indicate like elements.

### DETAILED DESCRIPTION

Delivery of therapeutics directly into the female reproductive tract (FRT) is beneficial for a number of reasons, including but not limited to the ability to avoid first pass metabolism in the liver, increased bioavailability, decreased required drug concentration, and/or lowered incidence of adverse effects and toxicity. One of the goals of the present disclosure is to improve patient compliance by providing new alternative delivery platforms that require less frequent dosing by offering sustained-release of agents (e.g., ≥ 1 month).

### Part A-Methods and Compositions for Treating Bacterial Infections

According to the World Health Organization (WHO), infection by *Chlamydia trachomatis* is the most commonly diagnosed bacterial STI, with global prevalence of 4.2% and 2.7% in females and males, respectively. Meanwhile, *Trichomonas vaginalis,* with 170 million yearly incident cases, constitutes one of the most significant STPs. In addition, bacterial vaginosis (BV), in which Gardnerella vaginalis ("Gv") is the predominant BV-associated bacteria (BVAB), causes a disruption in the diverse microbial communities that maintain host homeostasis and innate defense in the female reproductive tract (FRT). BV infection and recurrence affects over 30% of women.

Existing topical delivery platforms, including gels, intravaginal rings (IVRs) and IUDs, have their respective delivery hurdles. Gels are challenged with leakage from the vaginal cavity. While IVRs offer longer delivery durations and durability, coadministration of both hydrophobic and hydrophilic agents from one device has proven difficult, and the high temperature processing conditions often associated with IVR fabrication may prove challenging for the incorporation of probiotics or other biological moiety. IUDs require implantation by a health professional, and do not protect against STIs.

This disclosure provides antimicrobial compositions that can be used, for example, to prevent and treat vaginal STIs and/or BV. Exemplary delivery platforms include, without limitation, core-shell polymeric electrospun fibers, b) multilayer composites, or c) interwoven composites. Embodiments of the disclosure provide an intravaginal probiotic fiber insert to be used against a variety of bacterial infections, included but not limited to vaginosis and yeast infections, which also depend on a microbiota balance and lactic acid concentration in the reproductive tract. The antimicrobial compositions described herein can extend treatment duration, thereby minimizing administration frequency and offering prolonged activity and protection against pathogens *in vivo.*

In one embodiment, an antimicrobial composition includes electrospun fibers comprising a probiotic embedded therein. Electrospun fibers (EFs) offer an effective strategy to deliver health promoting agents to the FRT. EFs, as the platform to extend treatment and prevention applications, have the ability to encapsulate or co-encapsulate a variety of active agents (e.g. antibiotics, antivirals, proteins, genes, probiotics and/or prebiotics); enhance encapsulant stability and half-life of an active agent relative to delivery of free agent; lower the doses necessary to achieve efficacy; and prolong the delivery of active agents in the FRT. Additionally, EFs provide high surface area-to-volume ratio and biomass that can foster an amenable environment for prolonged probiotic growth and retention.

Electrospun fiber materials are known in the art and can be biodegradable or non-biodegradable polymers. Representative biodegradable fiber materials include, without limitation, PLGA, mPEG-PLGA, PVA, PEO, PVP. Representative non-biodegradable fiber materials include, without limitation, polycaprolactone (PCL). Hydrophilic or hydrophobic fiber material can be used. In some embodiments, composite fibers of both hydrophobic (e.g. PLGA) and hydrophilic fibers are used. In some embodiments, hydrophilic fibers alone are used. Electrospun fiber materials can be non-toxic, FDA-approved and readily translatable across a variety of biomedical and clinical applications.

In some embodiments, a dual-electrospun mesh architecture of PEO and PLGA can be generated as follows. About 2.5% to about 7.5% (e.g., about 5%) w/w PEO in MRS broth or water and about 10% to about 20% (e.g., about 15%) w/w PLGA in HFIP solvent can be introduced simultaneously at a mandrel and electrospun to deposit a mesh-like fiber composition. In some embodiments, the distance from each syringe needle tip to the mandrel is about 20 cm to about 30 cm (e.g., about 25 cm) for PEO and about 10 cm to about 20 cm (e.g., about 15 cm) for PLGA. In some embodiments, the PEO can be electrospun under conditions that include a flow rate of about 0.2 mL/hr to about 0.4 mL/hr (0.3 mL/hr) and a voltage of about 20 kV to about 30 kV (e.g., about 25 kV), while the PLGA can be electrospun under conditions that include a flow rate of about 0.2 mL/hr to about 0.4 mL/hr (e.g., 0.3 mL/hr) (for a 75:25 mixture), about 0.05 mL/hr to about 0.2 mL/hr (e.g., 0.1 mL/hr) (for a 50:50 mixture) or about 0.2 mL/hr to about 0.4 mL/hr (e.g., about 0.03 mL/hr) (for a 25:75 mixture) and a voltage of about 15 kV to about 22.5 kV (e.g., about 18 kV). The mesh-like fiber can include either La or Lcr as the probiotic at a concentration of about 5 × 10e6 CFUs per mg fiber to about 5 × 10e8 CFUs per mg fiber (e.g., about 5 × 10e7 CFUs per mg fiber).

In addition to acting as an antimicrobial against *Gardnerella vaginalis* and C. *trachomatis,* the compositions and methods described herein can be used as antimicrobials against a number of other microorganisms including, without limitation, *Neisseria gonorrhoeae, Atopobium vaginae, Mycoplasma hominis, Mycoplasma genitalium, Ureoplasma urelyticum, Mobiluncus* spp., *Prevotella* spp., *Streptococcus* spp., *Megasphaera* spp., *Dialiester* spp., *Eggerthella* spp., *Megasphaera* spp., *Sneathia* spp., and/or *Leptotrichia* spp. Infection of the urogenital tract by other microbes such as E. *coli, P. falciparum*, *Entamoeba histolytica, Trypanosoma* spp., *Leishmania* spp., *Toxoplasma* spp., and/or *Acanthamoeba* spp. also can occur, and the compositions and methods described herein can be used as antimicrobials against such microorganisms.

Probiotics are beneficial microorganisms (e.g., viruses, bacteria, protozoa, fungi) that can stabilize the healthy microbiota and prevent or inhibit the colonization and growth of pathogenic organisms and include a number of different *Lactobacillus* spp. (e.g., *L. acidophilus, L. crispatus, L. rhamnosus, L. reuteri, L. jensenii, L. gasseri*)*.*

Prebiotics are compounds that selectively target and stimulate the growth and/or activity of beneficial bacterial (e.g., probiotics) and include, without limitation, fructans (e.g., fructooligosaccharides (FOS), inulins), galactans (galactooligosaccharides (GOS)), pectin, beta-glucans, and xylooligosaccharides.

In some embodiments, an antimicrobial composition as described herein can further include an active agent (e.g., separate from the probiotic and accompanying prebiotics). For example, an active agent can be an anti-retroviral, an anti-viral (including other entry inhibitors or active agents with other mechanisms of action on viral life cycle), polysaccharides (such as carrageenan), other antiviral proteins, oligonucleotides, griffithsin, or combinations thereof.

Methods of making an antimicrobial composition are described herein, and include electrospinning the fiber material and a probiotic. A variety of parameters of the process can be optimized (e.g. spinning solution, bacteria concentration, media supplements) to enhance the incorporation and the viability of probiotics within the fibers. As described herein, the antimicrobial compositions described herein can provide a long-term sustained release of probiotics.

The antimicrobial compositions described herein can be used in a number of different applications including, for example, methods of improving the endogenous vaginal microflora in the reproductive tract of a female, methods of inhibiting the growth and/or reducing the amount of BV-associated bacteria (BVAB) in the reproductive tract of a female, and/or methods of treating a bacterial infection in the reproductive tract of a female. Such methods require implanting an antimicrobial composition as described herein into the reproductive tract of the female. The probiotic bacteria are able to proliferate within the fiber, allowing sustained-release of the probiotic for days, weeks, or months.

The intravaginal delivery platforms described herein can be dosed, for example, at 10e8 to 10e10 CFU/day or an amount sufficient to produce, or increase the production, of lactic acid, thereby changing the pH of the female reproductive tract. The antimicrobial compositions described herein provide significant beneficial effects as they can provide a sustained release of the probiotic into the reproductive tract of the female, which can require a reduction in the frequency of administration.

### Part B-Methods and Compositions for Treating Viral Infections

There are a number of antiretrovirals (ARVs) available for the treatment of HIV-1 (e.g., Tenofovir (TFV), Raltegravir (RAL) and Dapivirine (DAP)), which can be classified based on their mechanism of action. Oral pre-exposure prophylaxis (PrEP) has demonstrated effectiveness for HIV prevention, but user adherence is critical to maintain effectiveness. In addition, renal and bone toxicity associated with long-term use, and increased risk of viral resistance are major concerns. Further, adverse effects can be experienced due to the poor oral bioavailability and frequent dosing required of many of these drugs. Thus, the high risks of drug resistance and long-term side effects limit ARV use.

As discussed herein, electrospun fibers (EFs) provide a stationary scaffold that can be used to encapsulate or embed one or more active agents. Electrospun fibers can be useful in the sustained-release of an active agent.

In addition, nanoparticles (NPs) can dramatically enhance the transport of biologics and other active agents, and improve delivery of biologics. NPs can reduce the need for frequent dosing, and typically elicit a minimal immune response in the FRT.

In one embodiment, an antiviral composition includes electrospun fibers comprising griffithsin-encapsulated nanoparticles embedded therein. In another embodiment, an antiviral composition includes electrospun fibers comprising griffithsin embedded therein.

As discussed herein, electrospun fiber materials are known in the art and can be biodegradable (e.g. PLGA, mPEG-PLGA, PVA, PEO, and/or PVP) or non-biodegradable (e.g., polycaprolactone) fibers and PLGA or mPEG-PLGA nanoparticles. As described herein, the nanoparticles incorporate griffithsin (GRFT), and provide efficacious, and safe sustainable-release of the GRFT. In some embodiments, mPEG-PLGA nanoparticles containing about 100 µg of GRFT can be synthesized using a double emulsion solvent evaporation technique. About 1% to about 20% of GRFT-encapsulated NPs can be incorporated into about 5% w/w PEO, which can be embedded within electrospun fibers that include about 10% to about 12% w/w polycaprolactone. In some embodiments, the mandrel-to-syringe distance can be about 10 cm to about 20 cm (e.g., 15 cm), the flow rate can be about 1.5 mL/hr to about 3 mL/hr (e.g., 2.2 mL/hr), and the voltage can be about 15 kV to about 25 kV (e.g., 20 kV). If desired, NP-PEO fiber sheets can be placed on the freshly electrospun PCL layer and an additional PCL layer can be electrospun on top to create a PCL-(NP/PEO)-PCL multilayered structure.

Griffithsin (GRFT) is a naturally-occurring lectin originally isolated from a marine red alga (Griffithsia sp.), that has exceptionally potent anti-viral activity within the mid-picomolar range. For HIV-1, GRFT is believed to bind to gp120 or mannose-linked glycans at entry; for HSV-2, GRFT is hypothesized to inhibit infection via glycoproteins expressed during cell-to-cell spread. Moreover, GRFTs success in inhibiting HIV and other viruses such as HSV-2, both in vitro and in vivo, highlights GRFT's potential as a multipurpose agent. Additionally, because it is a small protein, and not orally bioavailable, it is unlikely that GRFT will be developed as part of the normal ARV treatment regimen, making it available for prophylaxis without compromising population-level ARV efficacy. Lastly, synergistic efficacy between GRFT and traditional ARVs has been demonstrated in preventing both cell-free and cell-associated virus transmission, suggesting the combination of multiple mechanistic agents in future work.

As an active pharmaceutical ingredient (API), GRFT has been thoroughly assessed for safety and efficacy against several enveloped viruses (e.g., HIV-1; Hepatitis C virus (HCV), severe acute respiratory syndrome coronavirus (SARS-CoV), and HSV-2) in preclinical studies. Recently, an oxidation-resistant variant of GRFT was developed, in which the Met at position 78 was replaced by a Gln. The resulting variant, referred to as Q-GRFT, was shown to retain the biophysical, anti-viral and safety properties of wild-type GRFT, with no susceptibility to oxidation, making it a more suitable HIV microbicide candidate. In addition, Q-GRFT with modifications to one or more amines for easier labeling can be used.

As described herein, multilayer fiber constructs, comprised of hydrophobic PCL layers surrounding an inner hydrophilic polymer encapsulating GRFT NPs, provide tunable release of GRFT. A variety of constructs and polymers for both fiber and nanoparticle fabrication have been developed, which show promising in vitro efficacy and release profiles, and these platforms will prolong GRFT efficacy against multiple STIs.

In some embodiments, an antiviral composition as described herein can further include an active agent (i.e., other than griffithsin). For example, an active agent can be a non-griffithsin anti-retroviral, non-griffithsin antiviral (including other entry inhibitors or active agents with other mechanisms of action on viral life cycle), polysaccharides (such as carrageenan), other antiviral proteins, probiotics, prebiotics (e.g., glycogen derivatives, protein sources), oligonucleotides, etc.

Methods of making an antiviral composition are described herein, and include electrospinning fiber material and griffithsin-encapsulated nanoparticles into electrospun fibers having the griffithsin-encapsulated nanoparticles embedded therein. As described herein, a variety of parameters of the process can be optimized (e.g. spinning solution, nanoparticle concentration, media supplements) to enhance the incorporation and the viability of griffithsin-encapsulated nanoparticles within the fibers.

The antiviral compositions described herein can be used in methods of treating a viral infection in the reproductive tract of a female. As described herein, such methods require implanting an antiviral composition as described herein into the reproductive tract of the female. As described herein, implanting certain of the antiviral compositions described herein provides a sustained release of the griffithsin into the reproductive tract of the female that can approach and exceed about 90 days. Also as described herein, implanting certain of the antiviral compositions described herein provides a rapid-release of the griffithsin into the reproductive tract of the female (e.g., hours or days or weeks).

In accordance with the present invention, there may be employed conventional molecular biology, microbiology, biochemical, and recombinant DNA techniques within the skill of the art. Such techniques are explained fully in the literature. The invention will be further described in the following examples, which do not limit the scope of the methods and compositions of matter described in the claims.

### EXAMPLES

### Part A-Methods and Compositions for Treating Bacterial Infections

### Example 1-Fabrication and Characterization of Electrospun Fibers that Incorporate Probiotics Against Bacterial Vaginosis Infection

### Introduction

Bacterial vaginosis (BV) is a polymicrobial imbalance of the vaginal microbiota associated with reproductive infection, preterm birth, and other adverse health outcomes. It affects over 30% of women at a given time. In the normal vaginal microbiota, *Lactobacilli* are the primary microbial community, which maintains host homeostasis and provides innate defense against incoming pathogens. However, for women afflicted with BV, *Gardnerella vaginalis* (Gv) is one of the most frequently isolated species contributing to BV. While the current treatments for BV include orally- and topically-applied antibiotics, administered in the form of tablets, creams, or gels, BV recurrence and drug-resistance with chronic antibiotic usage are the main challenges of current treatment strategies.

### Objective

The goal of this study was to develop and characterize the hydrophilic core of novel polymer core-shell electrospun fibers (EFs), to highly load and release the probiotic, *Lactobacillus acidophilus* ("La"). In addition, we sought to determine the ability of La EFs to decrease the viability of Gv in a liquid co-culture assay. This study provides composite sustained-release probiotic fibers that decrease Gv viability and biofilm formation in the female reproductive tract.

### Methodology

Bacteria were prepared with appropriate medium at 37°C in an anaerobic environment. Polyethylene oxide (PEO, 600 kDa), polyvinyl alcohol (PVA, 146-186 kDa) and polyvinylpyrrolidone (PVP, 1.3 MDa) were electrospun with viable La (5 × 10e6 to 5 × 10e8 CFU per mg fiber).

Bacteria loading, viability, and stability were tested by CFU count, BacLight live/dead assay, and fluorescence microscopy. La viability and stability were evaluated at -20°C, 4°C, and 25°C by counting colony forming units (CFUs). To determine the ability of probiotic EFs to decrease Gv viability, a La EF-Gv co-culture was assessed.

### Results

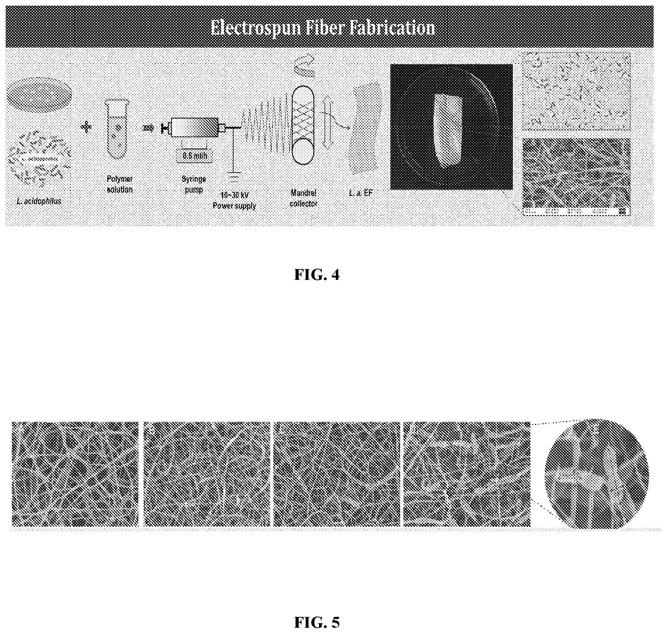

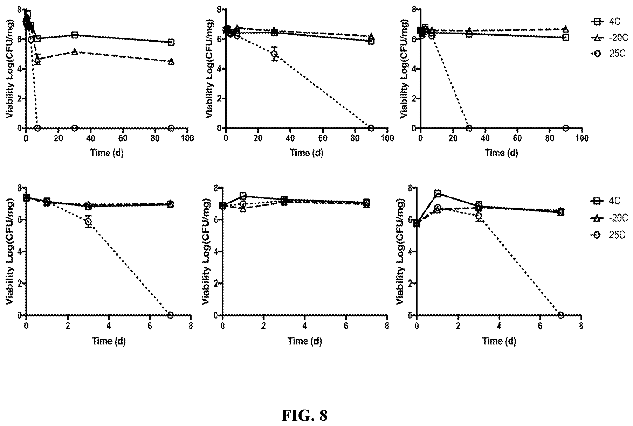

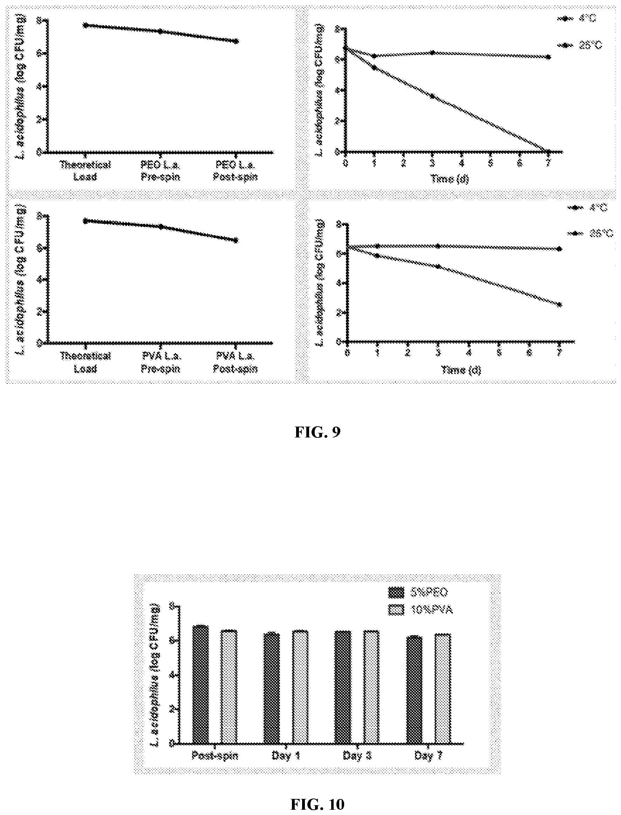

FIG. 5 shows SEM images of 5% PEO electrospun fibers containing different concentrations of *L.* acidophilus, and FIG. 6 shows SEM images of 5% PEO electrospun fibers containing *L. acidophilus* in different formulations. FIG. 7 shows the viability and stability of *L. acidophilus* in electrospun PEO fibers at different time points, and FIG. 8 shows the viability and stability of *L. acidophilus* in different electrospun PEO and PVA fiber formulations at different time points. FIG. 9 shows the viability of La in 5% PEO or 10% PVA fibers. Over 5 × 10e6 CFU/mg were recovered from the EFs. FIG. 10 shows a comparison of La viability between PEO and PVA fibers at 4°C. High variability was maintained at 4C, and no statistically significant differences were observed between PEO and PVA La fibers.

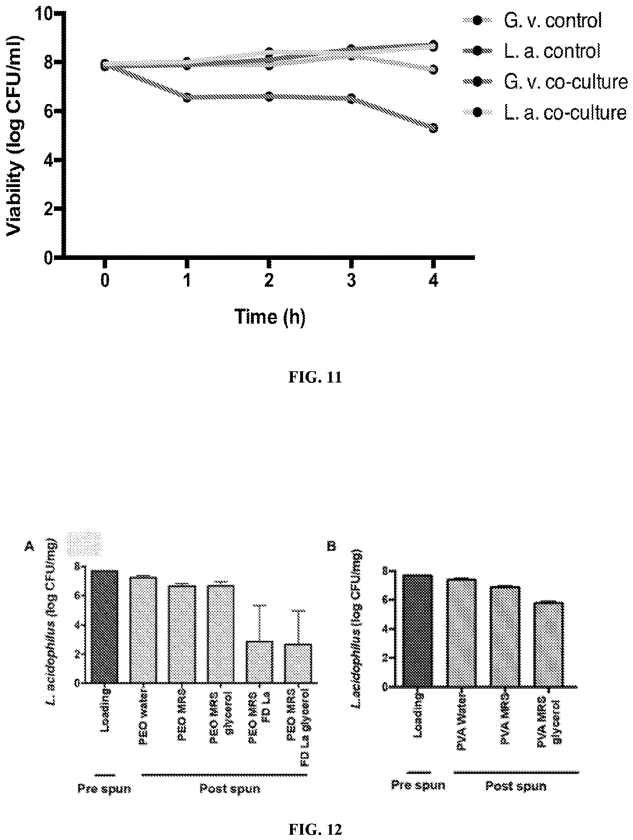

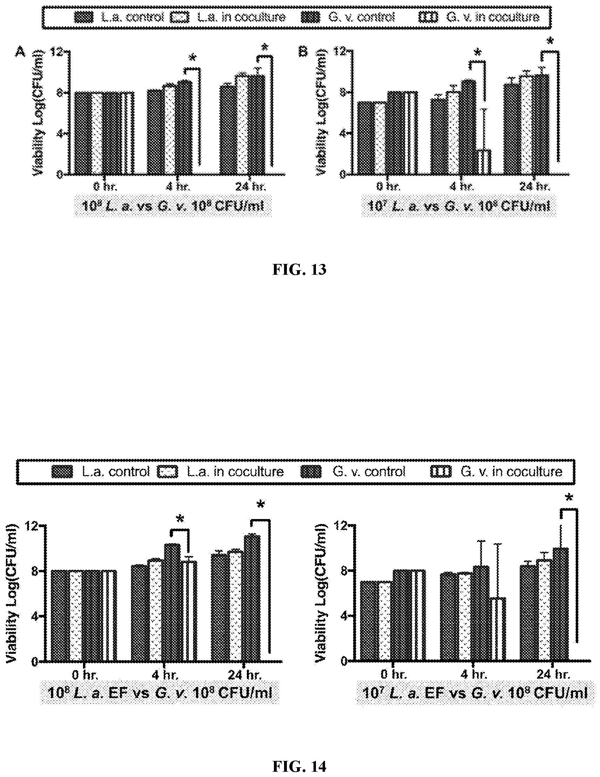

FIG. 11 shows the results of co-culture of free La and Gv. A significant decrease in clinically isolated Gv viability was seen after 4 hr co-incubation of La-Gv co-culture. FIG. 12 shows the La viability in different PEO and PVA formulations after electrospinning as a function of CFU per mg fiber. The amount of bacteria loaded in polymer solution prior to electrospinning, determined via CFU counts, was considered 100% viable. FIG. 13 shows the results of co-culture with free La and Gv. La EFs demonstrated inhibition of Gv growth 4 and 24 hr after incubation.

### Conclusions

This study provided the foundation to improve therapeutic outcomes of BV using a novel EF delivery vehicle, and to understand the effect of probiotic fibers on the vaginal microbiome. These results demonstrate that *Lactobacilli* maintain high viability after electrospinning, and that *Lactobacilli* also can decrease the viability of Gv *in vitro.* No significant La viability (CFU/mg) difference was observed between PEO and PVA fibers.

As shown herein, different architectures of hydrophilic-hydrophobic fiber platforms can be used to provide sustained-release and greater longevity and viability of probiotics.

### Example 2-Determining the Effects of Prebiotics on Lactobacilli and Gardnerella vaginalis in Co-Culture

The goal of this project was to evaluate the impact that prebiotics have on free probiotic viability and growth, for future application in electrospun fibers. It was hypothesized that electrospun fibers (EFs) embedded with probiotics can be used intravaginally to improve viability and sustained prebiotic delivery to the reproductive tract.

### Methods

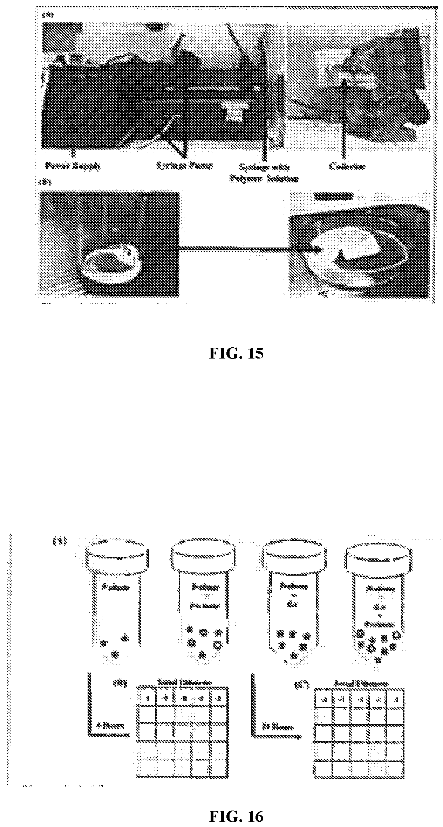

A fiber electrospinner (FIG. 15A) was used to fabricate polymer fibers from a polymer solution formulated prior to spinning, and fibers were formed via electrospinning, composed of PEO, probiotic, and prebiotic (FIG. 15B).

Bacteria were co-cultured in media using the following experimental design (FIG. 16A). The probiotic (*L. acidophilus* (La) or *L. crispatus* (Lcr)), the prebiotic, and the Gv were combined for bacterial cell co-culture experiments. Each tube was serially diluted and plated after 4 (FIG. 16B) and 24 (FIG. 16C) hours growth.

### Results

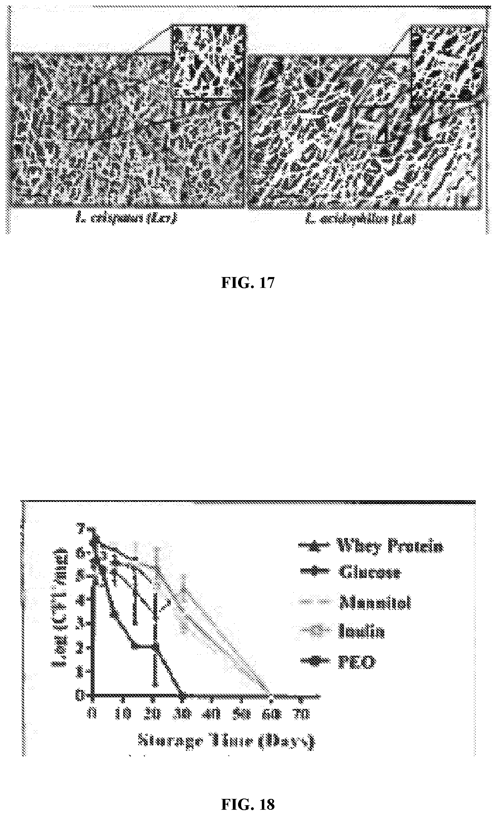

SEM images of the electrospun fibers containing Lcr (Left) and La (Right) were obtained (shown in FIG. 17 with inulin). In addition, the viability of the prebiotics in fibers also were evaluated under various conditions. For example, the viability of La probiotics in fibers stored in room temperature conditions for 60 days were evaluated (FIG. 18).

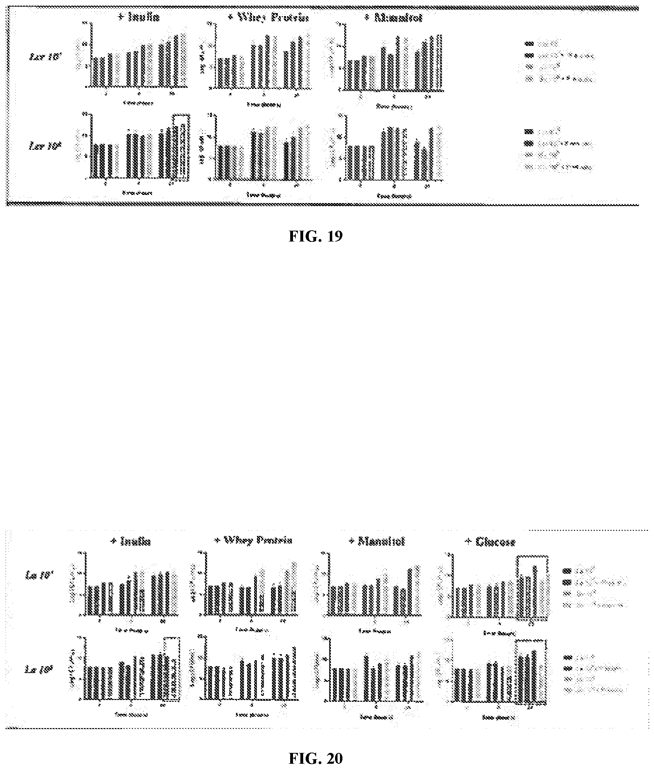

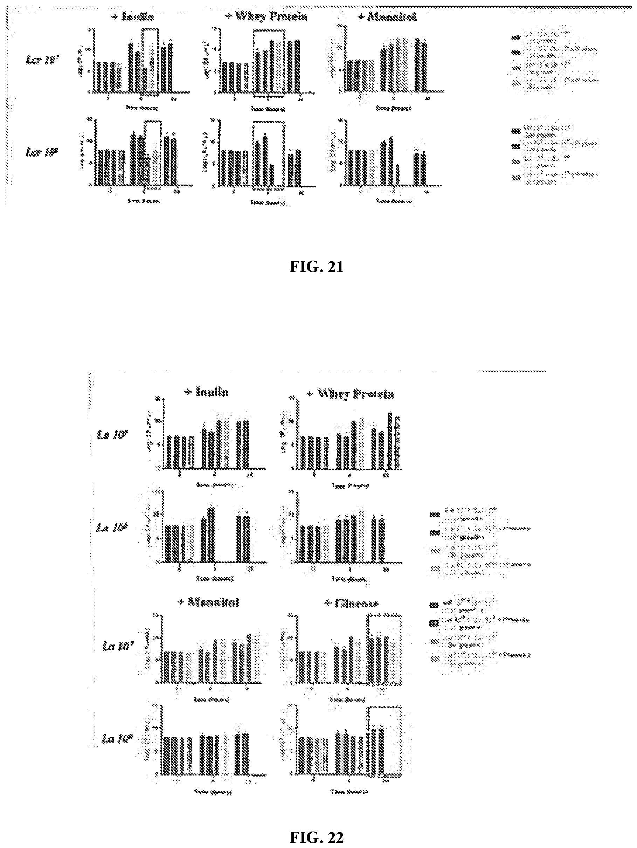

In addition, the viability of free Lcr (FIG. 19) or free La (FIG. 20) and Gv cultured alone with different prebiotics was evaluated, and the viability of free Lcr (FIG. 21) or free La (FIG. 22) and Gv in co-culture conditions with different prebiotics also was evaluated.

### Conclusions

Comparing Lcr 10e8 and La 10e8 in co-culture with inulin demonstrated that La allows for a decrease in Gv growth.

The comparison between Lcr 10e7 and Lcr 10e8 co-cultured with Gv and whey protein demonstrated that a higher concentration of probiotic inhibits the growth of Gv, and allows for increased probiotic growth.

Comparing Lcr 10e7 and Lcr 10e8 in co-culture with Gv and inulin demonstrated that there was an increase of Gv growth, which suggests that inulin may not be an ideal prebiotic.

It also was observed that glucose inhibited Gv growth and allowed for a steady or increased amount of probiotic growth, which suggests that glucose may be a much better prebiotic.

Hydrophilic rapid-dissolve and hybrid-blended electrospun fibers that contain a probiotic and a prebiotic are evaluated in co-culture experiments with Gv.

Probiotic electrospun fibers that contain prebiotic are evaluated with HeLa cells.

### Example 3-Developing a Multipurpose Prevention Technology for Bacterial and Parasitic Infections

The purpose of these experiments were to evaluate the impact Q-GRFT and Q-GRFT EFs and NPs have in repressing the growth of representative sexually transmitted bacterium (*C*. *trachomatis*) and protozoa (*T. vaginalis*) in cervicovaginal cell lines.

Free Q-GRFT is initially tested for its binding to surface glycoproteins, e.g. MOMP of C. trachomatis, by ELISA and microscopy. Growth inhibition assays are conducted to determine the minimal inhibitory concentrations of free Q-GRFT, Q-GRFT NPs, and Q-GRFT EFs or eluates against C. *trachomatis* and *T. vaginalis, in vitro.* Next, the effects of Q-GRFT and Q-GRFT NP and EF formulations on C. *trachomatis* and *T. vaginalis* adherence to host cells are assessed, and the ability of Q-GRFT products to inhibit host cell cytotoxicity induced by either pathogen is evaluated.

### Determine the Binding of Q-GRFT to C. trachomatis and T. vaginalis

Alexa-Fluor 488-labelled Q-GRFT is incubated with C. trachomatis (strain UW-43/Cx) or T. vaginalis (strain B7RC2) cultures for different durations ranging from 5 min to 6 hr. At each time point, cells are centrifuged, and the pellets are collected and washed to remove unbound Q-GRFT. Samples are examined for binding using fluorescence microscopy. Purified Alexa-Fluor 488 is used as a negative control, while a commercially available anti-MOMP or anti-*T*. *vaginalis* monoclonal antibody is utilized as a positive control to assess the binding of *C*. *trachomatis* EBs or *T. vaginalis* to Q-GRFT as previously described. Binding is validated by assessing fluorescence relative to a known labeled Q-GRFT standard using a microtiter plate reader.

### Determine the Effects of Q-GRFT on C. trachomatis and T. vaginalis Adherence to Cervicovaginal Epithelial Cells

Adherence of microorganisms is an important first step in microbial infection. Here, the well-characterized cervicovaginal cell lines, End1/E6E7, Ect1/E6E7, and VK2/E6E7 are employed to determine the ability of Q-GRFT to inhibit pathogen adherence to epithelial cells. Vaginal epithelial cells are first incubated with free Q-GRFT or Q-GRFT NP or EF formulations for 30-60 min prior to C. trachomatis UW-43/Cx or T. vaginalis B7RC2 exposure. A variety of free Q-GRFT, Q-GRFT NP, and Q-GRFT EF or eluate concentrations corresponding to 1-100 µg/mL GRFT are tested. After 0.5 - 2 hr infection, the epithelial cells are washed three times and cells are stained with fluorescein isothiocyanate (FITC)-labelled Chlamydiaspecific anti-lipopolysaccharide (anti-LPS) or anti-Trichomonas vaginalis monoclonal antibodies.

### Quantify the Minimal Inhibitory Concentration for T. vaginalis

The inhibitory effects of Q-GRFT and Q-GRFT NP and EF formulations are tested against several strains of T. vaginalis, including B7RC2, Hs4:NIH, and CDC 085, in both aerobic and anaerobic conditions. Serial dilutions of Q-GRFT, Q-GRFT NPs, or Q-GRFT EFs or eluates (between 1 and 100 µg/mL) are prepared in Diamond's trypticase-yeast extract-maltose (TYM) culture medium to culture T. vaginalis. The minimum inhibitory concentration is considered the concentration at which no viable cells are found, as assessed by light microscopy (differential count of motile to non-motile organisms). Alternatively, a fluorescence method based on the amount of resazurin salt reduced by T. vaginalis isolates can be used. Samples without T. vaginalis serve as a negative control, while untreated pathogens serve as a positive control for growth.

### Quantify the Minimal Inhibitory Concentration for C. trachomatis

The inhibitory effects of Q-GRFT and QGRFT NP and EF formulations are tested against several C. trachomatis strains, including UW-43/Cx and the mouse pneumonitis, Nigg II (ATCC# VR-123), to evaluate inhibitory potential. Q-GRFT, Q-GRFT NPs, or QGRFT EFs or eluates are diluted in growth medium at different concentrations (between 1 and 100 µg/mL), and 2 × 10e4 infection forming units (IFU) of chlamydial inoculum are added to each dilution, and incubated at 35°C for 15, 30 or 60 min. The treated inoculum is subsequently used to infect HeLa cells on cover slips. After 48 hr of incubation at 35°C in a humid environment, the cover slips are analyzed microscopically upon staining with specific anti-LPS as described for adherence assays. The minimal inhibitory concentration are defined as the lowest concentration required for complete inhibition of C. trachomatis.

### Evaluate the Effects of Q-GRFT on C. trachomatis and T. vaginalis Induced Cell Cytotoxicity

Both *C*. *trachomatis* and *T. vaginalis* can induce host cell cytotoxicity. Pathogenic *T. vaginalis* strains have been shown to cause contact-dependent host cell cytotoxicity, while intracellular pathogens like C. *trachomatis* have strategies to manipulate apoptotic pathways and replicate. Infection by C. *trachomatis* generally results in inflammasome activation, with subsequent induction of pyroptosis, a form of cell death that restricts pathogen replication.

The effects of Q-GRFT on pathogen-induced host cytotoxicity are determined using the MTT assay after 1-3 days incubation. For both organisms, multiplex immunoassays are used to assess the effects of Q-GRFT as well as Q-GRFT NPs, and Q-GRFT EFs or eluates on host cell induced immune effectors as a consequence of infection; defense molecules including TNF-alpha, IL-1beta, IL-6, and IL-8 are assessed, among others. Data analysis is performed by one-way ANOVA for multiple groups, and a t-test is used for group pair comparison. P < 0.05 is considered statistically significant.

### Results

Q-GRFT and Q-GRFT NPs/EFs impart some level of protection against C. *trachomatis* and *T. vaginalis* infections. Initial dose estimates are based on 1 µg/mL, altering the adherence of *T. vaginalis* to human epithelial cells and resulting in less infectivity of the parasite in mice. In addition to the above dose, Q-GRFT is tested as high as 100 µg/mL, which could increase inhibitory effects. The appropriate concentrations of QGRFT NPs and EFs, based on GRFT release, is evaluated in these experiments. A failure to have *in vitro* inhibition by at least 50% for either organism indicates no *in vivo* testing.

### Evaluate the Anti-Infective Properties of Q-GRFT Formulations Against T. vaginalis and C. trachomatis In Vivo

Interfering with C. *trachomatis* and *T. vaginalis* imparts protection in respective murine models of infection. BALB/c mice are treated with medroxyprogesterone acetate 7 days prior to infection by the pathogens, and an additional 2 days prior for *T. vaginalis.* BALB/c mice are treated with a single dose of prophylactic Q-GRFT experimental or control treatments 24 hr prior to inoculation with either *T. vaginalis* or *C*. *trachomatis.* A maximum product dose of Q-GRFT gel or GRFT NPs/EFs is tested based on the minimum inhibitory concentrations determined in the experiments above. For infection with C. *trachomatis,* the mice are vaginally challenged with 10e6 IFUs under anesthesia as proposed previously. For *T. vaginalis* infection, logarithmic growth phase *T. vaginalis* trophozoites is washed, re-suspended in TYM, and administered as a single vaginal instillation of 5 × 10e5 cells. Sustained infection is achieved by treatment with *Lactobacillus acidophilus* in estrogenized BALB/c mice.

The primary endpoint is infection burden, assessed up to 14 days post-infection. To comprehensively assess the protective effects of Q-GRFT in infected animals, genital tissues are evaluated (using histology, immune cell populations, gene expression, and cytokine production), in addition to blood cell composition and serum chemistry parameters. For each experiment, a group of untreated animals with no pathogen inoculation is included; all groups have a minimum of 5 mice (N=5) and the experiments are repeated two times. The animals are assessed 2, 7, and 14 days post-infection according to the endpoints defined below.

### Primary and Secondary Endpoints

Infection burden are quantified by counting pathogenic cells (T. vaginalis or C. trachomatis) in vaginal fluids collected at the time points defined above. In the case of C. trachomatis, cells are obtained from sucrose-potassium glutamate buffer (pH 7.2) swab fluid and grown in HeLa 229 cells, and assessed by indirect immunofluorescence microscopy as described previously. In addition, antibody responses to MoPn antigen in mouse blood are evaluated after C. trachomatis infection by determining both total plasma IgG for MoPn-specific antibodies as described. For T. vaginalis, the viscous vaginal washes are incubated overnight in TYI-S-33 medium containing 10% bovine serum to release the organisms; and the parasites are fixed and counted. In addition to antibody response and cell counts, blood is collected at sacrifice to determine blood cell composition and serum chemistry markers. Reproductive tissues are submitted to hematoxylin and eosin (H&E) and Masson's trichrome staining for histopathology, followed by gene expression studies using qRT-PCR to detect changes in genes relevant to related infections. Multiplex immunoassays are used to detect protein level changes in immune effectors. Such molecules will include IL-1beta, IL-2, IL-4, IL-6, IL-8, IFNgamma, and TNF-alpha. Data analysis is performed by one-way ANOVA (multiple groups) and t-test (group pairs). Statistical significance is set at p<0.05.

### Results

The formulations used herein have proven sustained- or inducible-release properties. Therefore, Q-GRFT products also inhibit or prolong the inhibition of T. vaginalis infection.

### Part B-Methods and Compositions for Treating Viral Infections

### Example 4-Dual Purpose Sustained Release Griffithsin Nanoparticle-Fiber Composites Against HAV-1 and HSV-2 Infections

### Polymers and Solvents

mPEG-PLGA (LG 50:50, 5,000 MW mPEG-50,000 MW PLGA) and PLGA (50:50, 0.55-0.75 dL/g, 31-57k MW) were purchased from PolySciTech (Lafayette, IN) and Lactel Absorbable Polymers (Cupertino, CA), respectively. Polymers including PCL (80,000 MW), PEO (600,000 MW), PVA (87-90% hydrolyzed, 30,000-70,000 MW), and PVP (1,400,000 MW) were purchased from Sigma Aldrich (St. Louis, MO). Trifluoroethanol (TFE) and dichloromethane (DCM) were purchased from Thermo Fisher (Waltham, MA). Other chemicals, including dimethyl sulfoxide (DMSO) and thiazolyl blue tetrazolium bromide (MTT) were purchased from Sigma Aldrich. Griffithsin stock solution (12.0 mg/mL, in PBS) was manufactured in *Nicotiana benthamiana* following methods previously described.

### Nanoparticle Synthesis

mPEG-PLGA and PLGA nanoparticles containing GRFT were synthesized using the double emulsion solvent evaporation technique. First, 100 mg PLGA or mPEG-PLGA were dissolved in 2 mL of DCM and incubated overnight. The next day, the polymer solutions were vortexed, and 200 µL of GRFT stock solution (25, 50, or 100 mg/mL in PBS) was added dropwise to synthesize NPs with a theoretical loading of 50, 100, or 200 µg GRFT/mg polymer. GRFT-polymer mixtures were sonicated and added to 2 mL of 5% PVA solution, sonicated again, and subsequently transferred to a beaker containing 50 mL of 0.3% PVA solution for 3 hr. The newly synthesized NPs were washed with Milli-Q water three times to remove residual PVA and solvent, followed by lyophilization and storage at -20°C until use.

To assess NP loading within the NP-EF composite, Coumarin 6 (C6) NPs were synthesized as previously described. Briefly, C6 was dissolved in DCM (15 µg C6/mg polymer or 15 mg C6 in 200 µL DCM), followed by the dropwise addition of 200 µL to the 2 mL polymer/DCM mixture. Afterward, the fabrication process of C6 NPs mirrored that of GRFT NPs, with sonication of the polymer-solvent mixture, addition to 2 mL of 5% PVA solution, and subsequent transfer to 50 mL of 0.3% PVA solution for 3 hr.

### NP Loading in Hydrophilic (Inner Layer) Fibers

Hydrophilic fibers, incorporating GRFT mPEG-PLGA NPs, were fabricated by first adding PEO (5% w/w), PVA (20% w/w), and PVP (20% w/w) to scintillation vials containing 1 mL Milli-Q water and were incubated overnight. Blank fiber controls were electrospun with a mandrel-to-syringe distance of 20 cm, flow rate of 0.25-0.60 mL/hr, and a voltage of 15-20 kV. To determine the loading capacity of NPs within the fibers, C6 NPs were electrospun in fibers at 1, 2, 10, or 20% w/w (NP/EF weight ratio). For GRFT NP-EF composites, PEO fibers were electrospun with 1 or 20% w/w GRFT NPs. Electrospinning parameters were adjusted for GRFT and C6 NP-EFs by changing the flow rate to 0.2 mL/hr and voltage to 20-25 kV.

### NP-EF Composite Synthesis

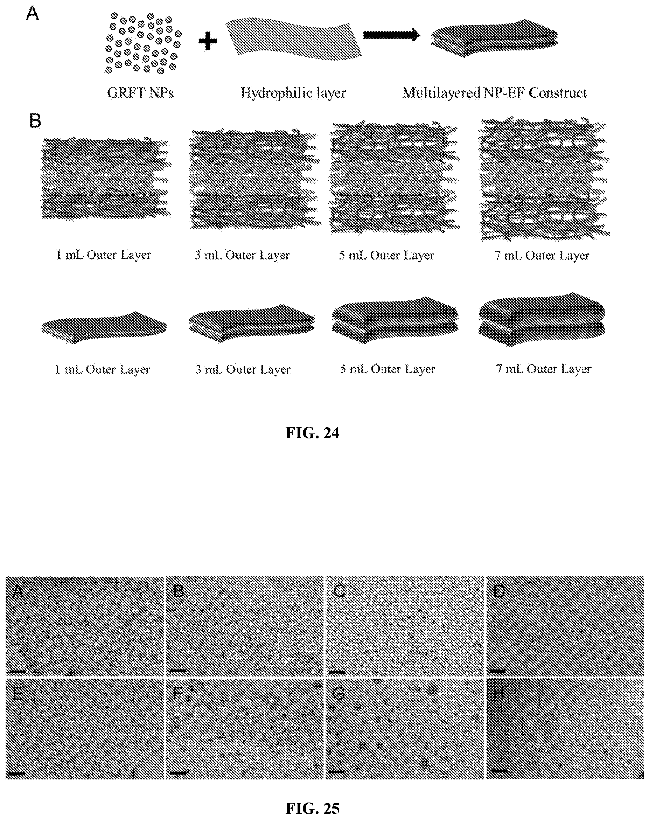

Polycaprolactone (11% w/w or 152 mg/mL) was dissolved in 7 mL TFE and incubated overnight. Different PCL solutions were electrospun using volumes of 1, 3, 5 or 7 mL to create composites with varying outer layer thicknesses. Similar electrospinning conditions were used with the mandrel-to-syringe distance set to 15 cm, flow rate of 2.2 mL/hr, and voltage of 20 kV. Pre-massed NP-PEO fiber sheets were placed on the freshly electrospun PCL layer and an additional PCL layer was electrospun on top to create a PCL-(NP/PEO)-PCL multilayered structure (FIG. 24), and fibers were desiccated overnight prior to characterization.

### NP and NP-EF Composite Morphology

Scanning electron microscopy (SEM) was used to characterize the physical structure and morphology of NPs, EFs, and NP-EF composites. Individual hydrophilic and hydrophobic fiber layers as well as multilayered composites were cut into 5 mm pieces and placed on double-sided adhesive carbon tabs (Ted Pella Inc., Redding, CA), which were then adhered to aluminum stubs. Nanoparticles were similarly adhered to carbon tabs and all samples were sputter coated with a thin gold alloy using a Bio-Rad E5100 sputter coat system (Hercules, CA). Samples of composite cross sections were prepared by slicing composites into 2 to 5 mm sections and adhering sections sideways to the aluminum stubs. Sample images were acquired in triplicate using a Supra 35 SEM (Zeiss, Oberkochen, Germany). Fiber and composite images were captured using an accelerating voltage of 8 kV, with magnifications of 5,000x to 10,000x, while NP images were captured with a voltage of 10 kV, and magnification of 20,000x to 23,000x. The mean diameters of NPs and fibers were determined using ImageJ software (NIH, Bethesda, Maryland), by measuring 50 line elements per SEM image.

### Quantification of GRFT NP, GRFT NP-EF, and C6 NP-EF Loading

The loading of GRFT in NPs and NP-EFs was assessed using an enzyme-linked immunosorbent assay (ELISA). Griffithsin loading and encapsulation efficiency (defined as [actual loading ÷ theoretical loading] × 100%) was determined for NPs and NP-EF composites. To determine loading, GRFT NPs and NP-EF composites (2 to 4 mg) were dissolved in 1 mL DCM, and vortexed for 30 s. Griffithsin was extracted from the DCM into 500 µL Tris-EDTA (TE) buffer, vortexed, and centrifuged at 18,500 x g for 5 min. TE buffer was collected, and the process was repeated to collect GRFT into a final volume of 1 mL. All extracts were analyzed using ELISA as previously described.

For the ELISA, 96-well Nunc Maxisorp plates were incubated overnight with 100 µL coating buffer (250 ng/mL gp120 in PBS). Coating buffer was removed from the plates, and 300 µL blocking buffer (0.05% Tween-20 and 3% w/v bovine serum albumin in PBS) was added to the wells for 2 hr. After incubation, the plates were washed with PBST (0.05% Tween-20 in PBS) using a Gardner Denver Multiwash III plate washer (Milwaukee, WI), and free GRFT standards (100 µL, 0.2 to 120 ng/mL) or sample extracts were added to each well. Plates were incubated for 1 hr at 37°C, followed again with a PBST wash. Dilutions of goat anti-GRFT primary antibody (1:10,000, kindly provided by Dr. Kenneth Palmer, University of Louisville) and rabbit anti-goat IgG-HRP secondary antibody (1:20,000, Sigma Aldrich) were subsequently added to each well in 100 µL volumes and incubated for 1 hr at 37°C to detect bound GRFT. After incubation, both antibody solutions were rinsed and 100 µL of KPL SureBlue TMB microwell peroxidase substrate (Sera Care, Milford, MA) was added to each well for 90 s, followed by quenching with 100 µL of 1 N H₂SO₄ (Thermo Fisher). Plate absorbance was measured at 450 nm on a Synergy HT reader (BioTek, Winooski, VT, USA) and data were analyzed using Prism (GraphPad Software, La Jolla, CA).

The loading of C6 NPs within NP-EFs was assessed via fluorescence. First, C6 NP-EFs (2 to 4 mg) were dissolved in 1 mL DMSO, and vortexed for 1 min to ensure complete composite dissolution. Composite dilutions were added (100 µL) to 96-well plates. C6 NPs (3 mg) were similarly dissolved in DMSO, and NP dilutions were used to generate a standard curve (ranging 6 ng/mL to 100 µg/mL), while dissolved blank NPs were used as background controls. Plates were read at excitation and emission wavelengths of 443 and 494 nm using a Synergy HT reader.

### Quantification of GRFT Release from NPs and NP-EF Composites

The sustained-release of GRFT from NPs and NP-EF composites was assessed for 30 and 90 d, respectively. GRFT PLGA and mPEG-PLGA NPs (3 to 5 mg) were aliquoted to 1.5 mL centrifuge tubes that contained 1 mL of simulated vaginal fluid (SVF), while NP-EF composites were placed in 10 mL scintillation vials and incubated in 3 mL SVF to ensure complete submersion. All samples were placed in a shaker at 37°C and 150 rpm, and at specific time points, the SVF was collected and replaced with fresh SVF. To collect NP release eluate, samples were centrifuged at 18,500 x g at 4°C for 10 min prior to supernatant collection.

### Cell Lines and Virus

TZM-bl cells, obtained from the National Institutes of Health AIDS Research and Reference Reagent Program (ARRRP), are engineered HeLa cells that express CD4, CCR5 and CXCR4 receptors, and were used to assess *in vitro* HIV-1 infectivity. TZM-bl cells are highly permissive to infection by most HIV-1 and pseudovirus strains, and contain HIV-activated Tat-driven luciferase, allowing for precise quantification of infection. TZM-bl cells were cultured in Dulbecco's Modified Eagle medium (DMEM) containing 10% fetal bovine serum (FBS), 25 mM HEPES, and 50 µg/mL gentamicin (Thermo Fisher).

The Env-pseudotype HIV-1 was produced by introducing HEK-293T/17 cells (ATCC, Rockville MD) with both an envelope (env)-expressing plasmid (CCR5-tropic clade A strain, Q769.h5) and an env-deficient HIV-1 backbone vector (pNL4.3ΔEnv-Luc). Both plasmids were obtained from the NIH AIDS Reagent Program (Catalog# 11884 and 3418). Cells were maintained in minimum essential medium (MEM) supplemented with 10% FBS, 1% penicillin (100 µg/mL), and 1% streptomycin (100 µg/mL) (VWR Radnor, PA).

HSV-2 infection was assessed in plaque assays in Vero E6 cells (African green monkey kidney). HSV-2 (4674) was kindly provided by Dr. Betsy Herold from Albert Einstein College of Medicine. Cells were maintained in MEM supplemented with 10% FBS, and 1% penicillin and streptomycin.

Finally, vaginal keratinocyte (VK2/E6E7), ectocervical (Ect1/E6E7), and endocervical (End1/E6E7) cell lines were used to assess fiber cytotoxicity (all cells originally from ATCC, Rockville MD). VK2/E6E7 (VK2), Ect1/E6E7 (Ect1), and End1/E6E7 (End1) are well-characterized immortalized cell lines derived from normal human vaginal, ectocervical, and endocervical epithelia, respectively, and are representative of the cell types found within the FRT. Cells were maintained in keratinocyte serum-free medium (KSFM) supplemented with recombinant human epidermal growth factor (0.1 ng/mL), bovine pituitary extract (50 µg/mL), calcium chloride (0.4 mM) (Thermo Fisher), with 1% penicillin and streptomycin. During cell trypsinization, plating, and cell counting, cells were neutralized with 1: 1 DMEM:Nutrient Mixture F-12 media (Thermo Fisher) with 10% FBS, and 1% penicillin/streptomycin.

### In Vitro HIV Inhibition

The activity of GRFT NPs and NP-EF composites against HIV-1 infection was assessed using an *in vitro* HIV inhibition assay as previously described. HIV inhibition was determined as a function of reduction in luciferase reporter gene expression after a single round of infection with pseudotyped HIV in TZM-bl cells. The optimal virus dilution (1:16) was established to yield ≥ 96,800 relative luminescence units (RLUs). Briefly, GRFT NP or NP-EF eluates were prepared in either PBS or DMEM (1.0 mg/mL), followed by 1:2 serial dilutions with DMEM to a final volume of 50 µL within a 96-well plate. One hundred microliters of TZM-bl cells (10,000 cells in DMEM supplemented with 10 µg/mL DEAE-dextran) was added to each well, followed by the addition 50 µL of HIV-1 pseudovirus dilution. Infected cells administered NPs or NP-EFs were incubated at 37°C for 48 hr and compared to the inhibition obtained with free GRFT-treated/infected (stock concentration 50 µg/mL), untreated/infected, and untreated/uninfected control groups.

After 48 hr, 100 µL of culture medium was removed from each well. Luminescence was measured using the Bright-Glo luciferase assay (Promega Corporation, Madison, WI) by adding 100 µL Bright-Glo reagent solution to each well for 5 min and recording relative luminescence units (RLUs, Synergy HT reader, BioTek). All RLU values were corrected by subtracting the RLU of untreated/uninfected cells from the RLUs of treated/infected cells. The percent virus inhibition was determined by normalizing the corrected RLUs of treated/infected cells to corrected untreated/infected cells with the following formula: % Infection = [(sample RLU - untreated uninfected cells) ÷ (untreated infected cells - untreated uninfected cells)] × 100%. The antiviral activity is reported as the half maximal inhibitory concentration (IC₅₀) and was calculated by comparing the sample luminescence intensity to the luminescence intensity from untreated/infected controls.

### In Vitro HSV-2 Inhibition

HSV-2 plaque assays were conducted to determine the *in vitro* efficacy of GRFT mPEG-PLGA NPs against HSV-2 infection. Briefly, Vero E6 cells were seeded at a density of 600,000 cells/well in a 6-well flat bottom plate and grown for 24 hr to complete confluence. Prior to cell infection, ~20 mg of GRFT NPs were added to 20 mL complete plating media (1% FBS MEM). Once the cells were fully confluent, the growth media was removed and replaced with 2 mL dilutions per well of GRFT NPs, followed by the introduction of HSV-2 (10 µL containing 3,000 PFU per well) after 1 hr of treatment. Free GRFT (2,000 µg/mL) corresponding to the concentration needed to provide complete inhibition and untreated/infected cells, were used as negative and positive controls of HSV-2 infection. Untreated/uninfected cells were used as additional negative controls.

After HSV-2 infection, plates were incubated for 48 hr at 37°C. After incubation, media was removed, cells were fixed for 10 min with 1.5 mL of methanol, stained with crystal violet (0.1% crystal violet in 80:20 water: ethanol solution), and incubated for 30 min. Finally, plates were washed with Milli-Q water, dried, and plaques were counted. Plaque numbers from experimental groups were normalized to the number of plaques in untreated/infected cells (~280-300 plaques). Samples were analyzed in triplicate, and GraphPad Prism software was used to determine and compare the half maximal inhibitory concentration IC₅₀ values of GRFT NPs to free GRFT.

### In Vitro Cytotoxicity

Vaginal epithelial (VK2/E6E7), ectocervical (Ect1/E6E7), and endocervical (End1/E6E7) cells were incubated with either blank NPs, blank NP-EFs, GRFT NPs, or GRFT NP-EF composites to assess the *in vitro* cytotoxicity of GRFT delivery platforms. Cells were plated at a density of 50,000 cells/well in 96-well plates and incubated in triplicate with either 0.5 or 1 mg/mL NPs in media. Composites were tested by incubating cells with 0.5 mg NP-EF pieces (2.5 mg/mL in media). No treatment (media alone) and 10% DMSO were used as positive and negative controls of cell viability, respectively. After 24, 48, and 72 hr incubation, 20 µL of MTT reagent was added to the cells and incubated for an additional 4 hr. Cells were lysed overnight with 100 µL lysis buffer (10% sodium dodecyl sulfate in 0.01 M hydrochloric acid). Absorbance values were measured at 570 nm the following day and normalized to untreated cell absorbance to obtain percent viability.

### In Vivo HSV-2 Efficacy

All *in vivo* experiments were approved by the University of Louisville's Institutional Animal Care and Use Committee (IACUC-17135) prior to testing. Animal studies were conducted using 5-week-old female BALB/c mice (Jackson Laboratory, Bar Harbor, ME) to evaluate the efficacy of mice to HSV-2 infection after administration of GRFT NPs and NP-EF composites (n=20). For these studies, mice were subcutaneously injected with 3 mg Depo-Provera (Revive, Madison, NJ) 5 days prior to vehicle administration to induce the diestrous stage of their cycle. All materials administered to mice were UV-sterilized for 15 s prior to use. Mice were administered 0.5 mg blank or GRFT NPs diluted in sterile PBS (20 µL of 25 mg/mL) or 20% w/w GRFT NP-EF composites (5 mg) under isoflurane anesthesia. Untreated/uninfected mice and mice treated with 20 µg free GRFT (20 µL of 1,000 µg/mL) served as negative controls of infection, while untreated/infected mice served as a positive control for infection. Blank mPEG-PLGA nanoparticles were administered as an additional control group in efficacy studies. A single challenge of HSV-2 LD₉₀ (here, 5,000 PFU, HSV-2(4674) was given 24 hr after vehicle administration, followed by 14 day observation using an established 4-point scale to monitor infection progression. Each day, mice were weighed and examined for neurological and epithelial damage. The study concluded 14 d after HSV-2 challenge, mice were euthanized, and survival was assessed. Kaplan-Meier survival curves were generated from the data, followed by log-ranked post hoc tests to assess the statistical significance between groups.

### In Vivo Safety

Additional studies were conducted to evaluate the safety of blank NPs and NP-EF composites *in vivo.* NPs and NP-EFs were UV-sterilized for 15 s, and intravaginally administered to mice under isoflurane anesthesia, at the same doses (0.5 and 5 mg, respectively) that were evaluated in the efficacy study. Control groups included uninfected mice that were untreated, treated with 20 µL free GRFT in PBS (1,000 µg/mL), or treated with 40 µL of N-9 gel. An additional "sham" control was used to evaluate the effects of tweezer administration alone. Mice were euthanized after 24 or 72 hr of vehicle administration (n=3 per time point) and reproductive tracts and vaginal lavages were collected and stored at -80°C following mouse euthanasia.

The structural integrity of the murine reproductive tracts was evaluated with histology. Collected tissue samples were washed with PBS, and subsequently fixed with 4% paraformaldehyde. The uterine horns, uterine body, and vagina were resected, fixed in 10% formalin, embedded in paraffin and sliced and stained with hematoxylin and eosin (H&E) for histological examination. Similarly, tissue samples and vaginal lavages were collected and analyzed for cytokine expression. The histology of sample cross-sections was assessed by a pathologist blinded to treatment groups.

Cytokine expression from murine reproductive tracts and vaginal lavages was determined using Luminex assays. First, 20 µL of T-Per solution (Thermo Fisher) containing 1% Halt Protease Inhibitor Cocktail (Thermo Fisher) was added per 1 mg of reproductive tissue. Approximately 20 zirconialsilica beads (BioSpec Productions) were added to each sample, followed by homogenization at 4,500x for 3 min (Bertin, France). Homogenized samples were cooled on ice for 5 min, and centrifuged at 10,000 x g at 4°C for 5 min. Sample supernatants were collected, aliquoted, and stored at -80°C for further study. Prior to Luminex analysis, interleukin 1 beta (IL-1β) levels were tested in the reproductive specimens using specific ELISA Ready-SET-Go! kits (Thermo Fisher) to confirm cytokine concentration ranges prior to the Luminex assays. Luminex assays were subsequently used to quantify cytokine levels, including G-CSF, INF-gamma, IL-1alpha, IL-1beta, IL-2, IL-6, IP-10, MCP-1, MIP-1alpha, MIP1-beta, MIP-2, and TNF-alpha from murine tissues and lavages. These cytokines were selected for assessment based on intravaginal inflammatory markers quantified in previous studies.

### Statistical Analysis

Unless otherwise noted, all *in vitro* experiments were conducted in triplicate, with 3 replicates per sample. Statistical analysis of samples for delivery vehicle morphology, loading, *in vitro* assays, and *in vivo* safety studies was performed by using one-way ANOVA with the Bonferroni post hoc test (p < 0.05). For murine efficacy studies, log-ranked post hoc tests were conducted to determine statistical significance as a function of treatment group and survival outcome.

### Nanoparticle Characterization

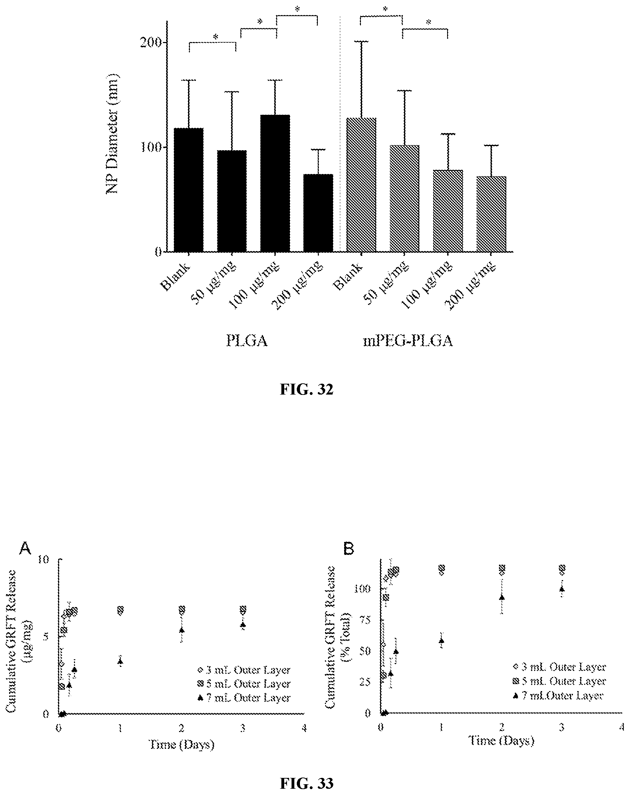

PLGA and mPEG-PLGA NPs were synthesized with 50, 100, or 200 µg GRFT per mg polymer. NPs were assessed for morphology, total GRFT loading, and sustained-release. The morphology and diameter of GRFT NPs, relative to blank NPs, were evaluated using SEM (FIG. 25) and ImageJ software (FIG. 32). All NP formulations possessed spherical morphologies, with diameters ranging from 72 to 131 nm. Blank nanoparticles demonstrated the largest diameters, with average PLGA and mPEG-PLGA NP diameters of 118 and 128 nm respectively. The addition of 50 µg/mg GRFT to PLGA and mPEG-PLGA NPs decreased NP diameters to 97 and 102 nm, respectively, while the addition of 100 µg/mg GRFT resulted in average diameters of 131 and 78 nm. The smallest PLGA and mPEG-PLGA NP diameters (72 and 74 nm), were achieved with the addition of 200 µg/mg GRFT. Overall the incorporation of GRFT corresponded to a decrease in NP diameter.

The loading of GRFT in PLGA and mPEG-PLGA NPs is shown in Table 1. The addition of 50, 100, and 200 µg GRFT/mg PLGA NPs resulted in 23.6 ± 1.4, 42.4 ± 3.1, and 77.8 ± 4.6 µg GRFT/mg PLGA respectively, while mPEG-PLGA NPs demonstrated consistently higher total GRFT loading of 49.6 ± 1.9, 63.6 ± 15.1, and 171.1 ± 21.5, for each formulation, respectively.

**Table 1. Griffithsin loading in mPEG-PLGA and PLGA NPs. Griffithsin extracts from NPs were assessed using ELISA. Increased theoretical loading resulted in increased actual loading. Three NP batches were fabricated for each formulation, with GRFT loading expressed as mean ± standard deviation of triplicate readings of each NP batch**

| Polymer | Theoretical Loading GRFT/NP (µg/mg) | Actual Loading GRFT/NP (µg/mg) | Encapsulation Efficiency (%) |
|---|---|---|---|
| PLGA NP | 50 | 23.6 ± 1.4 | 47.2 ± 2.9 |
| | 100 | 42.4 ± 3.1 | 42.4 ± 3.1 |
| | 200 | 77.8 ± 4.6 | 38.9 ± 2.3 |
| mPEG-PLGA NP | 50 | 46.6 ± 1.9 | 93.2 ± 1.7 |
| | 100 | 63.6 ± 15.1 | 63.6 ± 15.1 |
| | 200 | 171 ± 21.5 | 85.6 ± 11.0 |

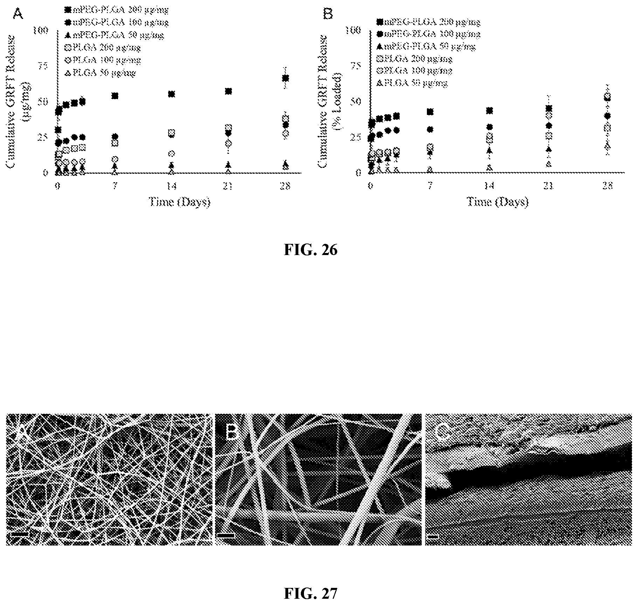

During NP release studies, an initial burst release was observed from both PLGA and mPEG-PLGA NPs through one week, followed by minimal GRFT release from 1 to 4 wk (FIG. 26). Increased release was seen from mPEG-PLGA NPs, relative to PLGA NPs, with 66.5 and 38.6 µg/mg GRFT released from 200 µg/mg mPEG-PLGA and PLGA NPs within 4 wk. Finally, as expected, the amount of GRFT release corresponded to the amount of GRFT loading for both polymers, with 200 µg/mg mPEG-PLGA NPs releasing the most GRFT over 4 wk.

### Nanoparticle-Electrospun Fiber Composite Characterization

Studies were conducted to assess the maximum loading that could be attained by adding C6 NPs to PEO, PVA, and PVP inner layer fibers. Fibers were loaded with 1, 2, 10 and 20% w/w NP/fiber, resulting in high NP incorporation, regardless of and corresponding to, theoretical loading (Table 2). The encapsulation efficiency of maximally-loaded NP-fiber composites (20% w/w) was 97, 65, and 46% for PEO, PVA, and PVP, respectively. Based on these results, PEO was selected as the hydrophilic layer for the multilayered composite.

**Table 2. Coumarin-6 NP loading in PEO, PVA, and PVP NP-EF composites. Dilutions of NP-EF composites, dissolved in DMSO, incorporating 10, 20, 100, or 200 µg/mg C6 NPs were evaluated by measuring fluorescence. Three composite batches were fabricated for each formulation, with C6 loading expressed as mean ± standard deviation of triplicate readings of each NP batch**

| Fiber Formulation | Theoretical Loading NP/EF (µg/mg) | Actual Loading NP/EF (µg/mg) | Encapsulation Efficiency (%) |
|---|---|---|---|
| PEO | 10 | 10.5 ± 1.2 | 105 ± 11 |
| | 20 | 15.8 ± 1.2 | 78.9 ± 3.9 |
| | 100 | 77.6 ± 23.0 | 77.6 ± 23.0 |
| | 200 | 194.6 ± 11.3 | 97.3 ± 6.0 |
| PVA | 10 | 7.4 ± 0.5 | 74.3 ± 4.3 |
| | 20 | 13.4 ± 1.2 | 67.3 ± 7.2 |
| | 100 | 86.0 ± 4.0 | 86.8 ± 4.9 |
| | 200 | 130.4 ± 42.0 | 65.2 ± 22.4 |
| PVP | 10 | 9.1 ± 1.1 | 91 ± 11.0 |
| | 20 | 9.2 ± 1.1 | 46.0 ± 6.2 |
| | 100 | 68.3 ± 7.2 | 68.3 ± 7.2 |
| | 200 | 147.2 ± 16.3 | 71.1 ± 8.2 |

Based on NP loading in the hydrophilic layer, multilayered PCL-PEO-PCL composites that incorporated 1 or 20% w/w mPEG-PLGA GRFT NPs (200 µg/mg GRFT) were fabricated. As shown in FIG. 27, both PEO (inner) and PCL (outer) layers maintained fiber morphology and integrity. Subsequent loading studies showed that 58 ± 8 and 56 ± 8% of GRFT NPs were incorporated from 1 and 20% NP w/w loaded multilayered NP-EFs.

Sustained-release studies were conducted to assess GRFT release from NP-EF composites with varying outer layer thicknesses corresponding to 1, 3, 5 and 7 mL volumes. First, release was measured from composites that contained 1% w/w GRFT NPs (FIG. 28A, 5B). All formulations, regardless of thickness, exhibited an initial burst release through ~72 hr, followed by sustained-release of GRFT for up to 90 d. Overall, increasing the PCL outer layer thickness resulted in decreased GRFT release. After 90 d, the total GRFT released from fibers was 81, 67, 54, and 57 ng/mg for 1, 3, 5 and 7 mL composites, respectively.

Based on the release results, composites that incorporated 20% w/w GRFT NPs with 1 and 3 mL PCL outer layers were fabricated. After 90 d, the total GRFT released was 11 and 9 µg/mg (53 and 45% total release) for the 1 and 3 mL NP-EF composites (FIG. 28C, 27D). Release profiles showed similar trends to 1% NP-EFs in that there was a noticeable burst release during the first 72 hr; however, decreased release was observed through week 4, while increased GRFT release was observed between weeks 4 and 8.

### In Vitro Viral Inhibition Studies

Both mPEG-PLGA GRFT NPs and NP-EF composites were assessed *in vitro* against HIV-1 and HSV-2 infections. Dilutions of GRFT NPs and eluates from 20% w/w NP-EF composites were administered to TZM-bl cells 1 and 24 hr prior to viral infection. GRFT NPs demonstrated complete dose-dependent inhibition against HIV-1 at both time points (FIG. 29A, 29B). Furthermore, the IC₅₀ of GRFT released from NPs was comparable to free GRFT. Additionally, GRFT released from NP-EF composites demonstrated comparable efficacy relative to free GRFT, achieving complete protection (> 98%) after 1 and 24 hr administration. The IC₅₀ values of GRFT NPs, GRFT NP-EFs, and free GRFT were 51.1 ± 2.3, 35.6 ± 7.1, 21.5 ± 5.3, after 1 hr and 36.1 ± 6.8, 29.7 ± 18.1, and 20.8 ± 6.7 ng/mL after 24 hr administration. All platforms demonstrated statistically similar IC₅₀ values to free GRFT, with the exception of 1 hr GRFT NPs, which showed a significantly higher IC50 value relative to both 1 and 24 hr free GRFT. These results indicate that incorporation of GRFT in either NPs or NP-EFs composites maintains antiviral activity.

### In Vitro Safety Studies

Prior to *in vivo* studies, both blank and GRFT NPs and NP-EFs were assessed for cytotoxicity in VK2/, Ect1/, and End1/E6E7 cells using MTT assays. All cell lines demonstrated greater than 94% viability after 24, 48, and 72 hr incubation with samples, relative to untreated controls (FIG. 29C - 29E).

### In Vivo Efficacy Studies

The efficacy of GRFT NPs and GRFT NP-EF composites was assessed within a murine model of lethal HSV-2 infection. Delivery platforms were intravaginally administered to female BALB/c mice, followed by a single challenge of HSV-2 (LD₉₀, 5,000 PFU) 24 hr later (FIG. 30A, 30B, Table 3). Mice were monitored for 14 d post-infection, and Kaplan-Meier survival analyses were applied to generate survival curves (FIG. 30C). Mice administered GRFT NPs or GRFT NP-EF composites showed statistically significant decreases in HSV-2 infection (70 and 80% survivability, respectively), relative to the untreated/infected control group (5% survivability, p < 0.05). Additionally, GRFT NPs exhibited statistically similar protection to free GRFT (85% survivability), whereas GRFT NP-EFs demonstrated a slight decrease in protection, relative to free GRFT controls (p < 0.05). Additionally, the administration of blank NPs provided a significant level of protection against HSV-2 (35% survival), relative to GRFT NPs, GRFT NP-EFs, and untreated/infected controls (p < 0.05).

**Table 3. Dosing regimen of GRFT NPs, GRFT NP-EFs, and free GRFT administered in in vivo studies**

| Formulation Name | Amount of Vehicle Administered per Mouse | Total GRFT Administered Based on Loading (ug) |
|---|---|---|
| GRFT NPs | 20 µL of 25 mg/mL PBS | 63 |
| GRFT NP-EFs | 5 mg | 71 |
| 0.1% w/v GRFT solution | 20 µL in PBS | 20 |

In addition to preventing HSV-2 infection, the mice treated with GRFT NPs, GRFT NP-EF composites, or free GRFT that became symptomatic, demonstrated delayed onset of symptoms and a more gradual progression of infection, relative to the untreated/infected control group (FIG. 30D-30G). In the untreated/infected control group, the first symptoms of HSV-2 infection typically manifest 4 to 5 d post-infection, during which time mice exhibit localized vaginal swelling and decreased hind leg mobility. After 5 to 8 d, -75% of mice from the untreated/infected control group required euthanasia due to the rapid progression and severity of infection. In contrast, mice exposed to free GRFT or GRFT delivery platforms showed decreased progression of symptoms within the same duration. Mice that did not survive HSV-2 challenge despite GRFT pre-treatment, exhibited a more gradual progression of infection, requiring euthanasia 7 to 10 d instead of 5 to 8 d post-infection, suggesting that GRFT may provide partial protection at these doses for the few mice that exhibited overt signs of infection. No differences were observed in the progression of HSV-2 infection after administration of blank NPs, relative to the untreated/uninfected control group.

### In Vivo Safety Studies

To assess the *in vivo* safety of these delivery platforms, blank NPs and NP-EF composites were intravaginally administered to uninfected mice, and reproductive tissue and vaginal lavages were collected and analyzed 24 and 72 hr following administration.

Tissue samples were evaluated for edema of muscle, interstitial, and epithelial tissue. In addition, the macrostructure of untreated and vehicle-treated cervical and vaginal epithelia were compared, and assessed for potential keratinization and goblet cell presence. A score ranging from 1 to 4 was used to determine the severity of epithelial changes. Images of tissues collected 24 hr after vehicle administration are shown in FIG. 31A - 31E. Extracted tissues from untreated controls demonstrated compact squamous and cervical epithelial tissue, with no evidence of edema nor inflammation. Similarly, tissues treated with blank NPs or NP-EF composites showed similar morphology and normality, relative to untreated tissue (scores of 1 to 2). Furthermore, no increase in lymphocyte accumulation was observed in these groups. Overall the tissues from vehicle-treated mice were comparable in morphology to tissues from untreated mice.

For N-9 treated mice, a slight increase in neutrophil accumulation was observed at the surface of the cervical squamous epithelium, relative to untreated controls, indicating minor topical damage (score range 1 to 2). One N-9 treated sample exhibited increased inflammation, due to the presence of peripheral blood mononuclear cells, goblet cell fusion, and epithelial disruption, resulting in a score of 3. However, the alterations observed in the N-9 control replicate were not observed in any 72 hr N-9 treated tissue controls, indicating transitory damage.

Finally, cytokine expression was assessed in collected murine tissues and vaginal lavages 24 and 72 hr after blank NP and NP-EF administration. Cytokine expression from blank NP or NP-EF treated mice was compared to levels in untreated mice, as well as to N-9 and sham-treated control groups. Cytokine expression was evaluated based on previously published guidelines, in which a significant level of intravaginal inflammation results in more than a 2 to 5-fold increase in cytokine expression, relative to untreated controls. Cytokine expression from extracted vaginal tissue and vaginal lavages is shown in FIG. 31F-31I, indicating that both blank NPs and NP-EFs are non-inflammatory. In both collected vaginal tissue and lavage samples treated with blank NPs and NP-EF composites, cytokine levels were comparable to untreated negative controls (p > 0.05). This trend was exhibited both 24 and 72 hr post-administration, with no statistical significance in cytokine expression between most untreated and blank fiber-treated groups. The only exceptions observed were in tissues that had been administered NPs for 72 hr, which demonstrated decreased cytokine levels of both MIP-1beta and TNF-alpha, and 72 hr wash samples from the NP-EF treated group, which showed increased IL-6 expression, relative to the untreated group. For sham-treated controls, which simulated the administration method via tweezers only, increased cytokine expression was observed in 24 hr tissue (MIP-2), 24 hr wash (IL-1beta), and 72 hr wash (IL-6, MCP-1), relative to untreated controls. The N-9 treated group demonstrated an increase of MIP-2 in 72 hr wash samples only. Both IP-10 and INF-gamma were undetected in vaginal lavage and tissue samples.

Increased TNF-alpha expression is typically associated with increased IL-1alpha, IL-1beta, and IL-2 expression, whereas increased MIP-1beta is associated with increased neutrophil accumulation, neither of which were observed in NP-treated tissue samples. Additionally, increased IL-6 expression alone is not indicative of inflammation, but is overexpressed with TNF-alpha and IL-1alpha under conditions of localized inflammation, which were not observed in NP-EF treated mice. Therefore, the increased expression of IL-6, MIP-1beta and TNF-alpha is attributed to variability in samples, and not inflammation, as the delivery platforms did not elicit increased expression of multiple cytokines. An ELISA, used to confirm IL-1beta levels, showed similar results, showing that both blank NP and NP-EF composites induced negligible increases in cytokine expression, relative to untreated controls (p > 0.05).

### Example 5-Dual-Purpose Rapid-Release Griffithsin Fibers for the Prevention of HSV-2 and HIV-2 Infections

### Materials

PEO (600,000 MW), PVA (87-90% hydrolyzed, 30,000-70,000 MW), and PVP (1,400,000 MW) were purchased from Sigma Aldrich (St Louis, MO). Organic solvents including dimethyl sulfoxide (DMSO) were also purchased from Sigma Aldrich. Cell culture media and reagents including Dulbecco's modified Eagle medium (DMEM), minimum essential media (MEM), fetal bovine serum (FBS), penicillin, streptomycin, and 4-(2-hydroxyethyl)-1-piperazineethanesulfonic acid (HEPES) were purchased from VWR (Radnor, PA). Keratinocyte Serum Free Media (KSFM) and gentamicin were purchased from Thermo Fisher (Hampton, NH).

### Cell Lines and Virus

TZM-bl cells, obtained from the National Institutes of Health (NIH) AIDS Research and Reference Reagent Program (ARRRP), were used to assess *in vitro* HIV-1 infectivity. TZM-bl cells are engineered HeLa cells that express CD4, CCR5 and CXCR4 receptors and contain a Tat-driven luciferase gene, which is activated by HIV-1 infection and permits sensitive and accurate measurements of infection. TZM-bl cells are highly permissive to infection by most strains of HIV and molecularly cloned Env-pseudotyped viruses. TZM-bl cells were cultured in DMEM containing 10% FBS, 25 mM HEPES, and 50 µg/mL gentamicin.

The Env-pseudotype HIV-1 was produced in house by transducing HEK-293T/17 cells with both an envelope (env)-expressing plasmid (CCR5-tropic clade A strain, Q769.h5) and an env-deficient HIV-1 backbone vector (pNL4.3ΔEnv-Luc). Both plasmids were obtained from the NIH AIDS Reagent Program (Cat# 11884 and 3418). HEK-293T (human embryonic kidney) cells were purchased from ATCC. Cells were maintained in minimum essential medium (MEM) supplemented with 10% FBS, penicillin (100 µg/mL), and streptomycin (100 µg/mL).

To conduct HSV-2 plaque assays, Vero E6 cells and HSV-2 (4674) were kindly provided by Dr. Kenneth Palmer from the University of Louisville. Cells were maintained in MEM supplemented with 10% FBS, penicillin (100 µg/mL), and streptomycin (100 µg/mL).

Finally, vaginal keratinocyte (VK2/E6E7), ectocervical (Ect1/E6E7), and endocervical (End1/E6E7) cell lines were used to assess fiber cytotoxicity (all cells courtesy of Dr. Kenneth Palmer, originally from ATCC, Rockville MD). VK2/E6E7 (VK2), Ect1/E6E7 (Ect1), and End1/E6E7 (End1) are well-characterized immortalized cell lines derived from normal human vaginal, ectocervical, and endocervical epithelia, respectively, which are representative of the cell types found within the FRT. Cells were maintained in KSFM supplemented with recombinant human epidermal growth factor (0.1 ng/mL), bovine pituitary extract (50 µg/mL), calcium chloride (0.4 mM), and 1% penicillin and streptomycin (100 µg/mL each). During cell trypsinization, plating, and counting, cells were neutralized with 1:1 DMEM:KSFM (with 10% FBS, and 1% penicillin/streptomycin (100 µg/mL each)).

### Rapid-Release Fiber Fabrication

Hydrophilic polymer solutions were fabricated by first weighing polymer into a glass scintillation vial and incubating overnight in 1 mL of Milli-Q water. To create PVA and PVP fibers with well-defined morphologies, 200 mg of either PVA or PVP were added to 1 mL Milli-Q water (20% w/v solution), while PEO fibers were fabricated by adding 50 mg of polymer to 1 mL Milli-Q water (5% w/v). Blank fibers were electrospun with a mandrel-to-syringe distance of 20 cm, flow rate of 0.2 to 0.3 mL, and a voltage of 15 kV. The flow rate and voltage were changed to 0.2 mL/hr and 25 kV for 1 and 10% w/w (GRFT to polymer weight ratio) GRFT fibers.

### Fiber Morphology

The morphology of blank PEO, PVA, and PVP fibers, as well as 1% and 10% GRFT w/w PEO, PVA, and PVP fibers was assessed using scanning electron microscopy (SEM). After electrospinning, fibers were dried for 24 hr in a desiccator, cut into 5 mm pieces and placed on double-sided adhesive carbon tabs (Ted Pella Inc., Redding, CA), which were adhered to aluminum stubs. The adhered fiber pieces were sputter coated with a thin gold alloy film using a Bio-Rad E5100 sputter coat system (Hercules, CA). The coating process was operated for 90 s at 20 mA. Multiple SEM images were acquired using a Supra 35 SEM (Zeiss, Oberkochen, Germany), with images captured under an accelerating voltage of 8 kV and using an average magnification of 1,000 to 5,000x. The average fiber diameter was determined with ImageJ software (NIH, Bethesda, MD), and a minimum of 50 fibers were assessed per image.

### Fiber Characterization

To assess GRFT loading, PEO, PVA, and PVP fibers were weighed (3 to 5 mg) into separate 1.5 mL Eppendorf tubes, followed by the addition of 1 mL PBS. After 1 to 2 min, the dissolved fiber solutions were vortexed and analyzed using ELISA to quantify GRFT loading and encapsulation efficiency (defined as: [actual loading ÷ theoretical loading] × 100).

The ELISA was conducted using 96-well Nunc Maxisorp plates as previously described. Briefly, plates were first prepared by coating wells with 100 µL of gp120 (250 ng/mL) in PBS, and incubating overnight at 4°C. Afterward, the coating buffer was removed and 300 µL of blocking buffer, consisting of PBS with 0.05% (v/v) Tween-20 (PBST) and 3% (w/v) bovine serum albumin, was added to each well. Plates were incubated at room temperature for 2 hr and then washed three times with PBST using a Multiwash III plate washer (Gardner Denver, Milwaukee, WI). One hundred microliters of GRFT standards (ranging from 0.2 to 120 ng/mL) and loading extracts were added to each well and incubated for 1 hr at 37°C. Dilutions of rabbit anti-GRFT primary antibody (1:10,000, provided by Dr. Kenneth Palmer, University of Louisville) and goat anti-rabbit IgG-HRP secondary antibody (1:20,000, Sigma-Aldrich) were added in volumes of 100 µL and each incubated for 1 hr at 37°C to detect bound GRFT. Finally, 100 µL of KPL SureBlue TMB microwell peroxidase substrate (Sera Care, Milford, MA) was added to each well for 90 s, and the reaction was quenched with the addition of 100 µL of 1 N H₂SO₄ (Thermo Fisher, Waltham, MA). Plate absorbance was measured at 450 nm on a Synergy HT reader (BioTek, Winooski, VT). Data were analyzed using Prism (GraphPad Software Version 6.0, La Jolla, CA).

### In Vitro HIV-1 Pseudovirus Inhibition Assay

The antiviral activity of GRFT fibers against HIV-1 was measured using an *in vitro* HIV-1 pseudovirus inhibition assay. As previously described, HIV-1 inhibition was determined as a function of reduction in luciferase reporter gene expression after a single round of infection in TZM-bl cells. The optimal virus dilution was established prior to the experiments to yield ~100,000 relative luminescence units (RLUs).

Briefly, 1% GRFT fibers (-3 to 5 mg) were first dissolved in sterile PBS, followed by serial dilutions (1:2) with DMEM to a final volume of 50 µL within a 96-well plate. TZM-bl cells (10,000 cells in 100 µL DMEM medium with 10 µg/mL DEAE-dextran) were subsequently added to each well, followed by the addition of 50 µL of diluted HIV-1 pseudovirus. Cells were then incubated at 37°C for 48 hr. Dilutions of free GRFT (stock concentration 50 µg/mL) ranging from 15 pg/mL to 120 ng/mL were similarly prepared for comparison.

After 48 hr, 100 µL culture medium was carefully removed from each well. Luminescence was measured using the Bright-Glo luciferase assay system (Promega Corporation, Madison, WI) by adding 100 µL Bright-Glo reagent solution to each well for 5 min. Plates were read via luminescence by the Synergy HT reader (BioTek). All RLU values were corrected by subtracting the RLU of untreated/uninfected cells from the sample RLUs (treated/infected cells). The percent virus inhibition was determined by normalizing the corrected RLUs of treated/infected cells to corrected untreated/infected cells: % Infection = [(sample RLU - untreated/uninfected cells) ÷ (untreated/infected cells - untreated/uninfected cells)] × 100. The antiviral activity of GRFT fibers is reported as the half maximal inhibitory concentration (IC₅₀), which was calculated by comparing the untreated/infected corrected control RLUs to the corrected RLU values of sample dilutions.

### In Vitro HSV-2 Plaque Assay

HSV-2 plaque assays were conducted to evaluate the *in vitro* efficacy of 10% w/w GRFT fibers against HSV-2 infection as previously described. Briefly, Vero E6 cells were seeded at 600,000 cells/well in a 6-well flat bottom plate (50% confluence) and grown for 24 hr to confluence. Prior to cell infection, 10% w/w GRFT fibers (30 mg) were dissolved in 20 mL complete plating media (1% FBS MEM). Once the cells were fully confluent, the growth media was removed and replaced with 2 mL of GRFT fiber eluate dilutions, followed by HSV-2 infection (3,000 PFU per well) 1 hr later. Free GRFT (2,000 µg/mL), corresponding to the concentration necessary to provide complete HSV-2 inhibition, was used as a positive control for inhibition, in addition to untreated/uninfected cells. Untreated/infected cells, were used as a negative control of inhibition.

Subsequent to HSV-2 infection, plates were incubated for 48 hr at 37°C, all media was removed, and cells were fixed with 1.5 mL methanol for 10 min. Afterward, 0.1% crystal violet was applied for 30 min to stain the plaques. Finally, plates were washed with Milli-Q water, and plaques were counted after drying. Plaque numbers from experimental groups were normalized relative to the number of plaques in untreated/infected cells (-280-300 plaques per well). Samples were analyzed in triplicate, and GraphPad Prism software was used to determine and compare the IC₅₀ values of GRFT fibers to free GRFT.

### In Vitro Cytotoxicity

Vaginal epithelial (VK2/E6E7), ectocervical (Ect1/E6E7), and endocervical (End1/E6E7) cells were administered either blank or GRFT fibers to assess *in vitro* safety. Cells were plated at a density of 50,000 cells/well in 96-well plates and incubated in triplicate with 0.5 mg fiber pieces placed in the solution (2.5 mg/mL final concentration). No treatment (media alone) and 10% DMSO were used as positive and negative controls of cell viability, respectively. After 24, 48, and 72 hr, 20 µL of MTT reagent was added to the cells and incubated for an additional 4 hr, followed by overnight lysis with the addition of 100 µL lysis buffer (10% sodium dodecyl sulfate in 0.01 M hydrochloric acid). Absorbance measurements (570 nm) were taken the following day. All sample absorbance values were normalized to untreated cell absorbance to obtain percent viability.

### In Vivo Efficacy Against a Lethal Dose of HSV-2 Infection

All *in vivo* experimental procedures were approved by the University of Louisville's Institutional Animal Care and Use Committee (IACUC 17135) prior to testing. All animal studies were conducted using 5-week-old female BALB/c mice (Jackson Laboratory, Bar Harbor, ME) to evaluate the efficacy and safety of GRFT fibers. For efficacy studies, mice were administered either blank or GRFT fibers (5 mg) that were UV-sterilized for 15 s. Mice were subcutaneously injected with 3 mg Depo-Provera (Revive, Madison, NJ) to induce the diestrous stage of their cycle, 5 days prior to fiber administration.

To determine the efficacy of GRFT fibers against HSV-2 infection, mice were administered a single dose of either GRFT PEO, PVA, or PVP fibers, or control groups (n=20). Twenty-four hours after fiber administration, mice were challenged with HSV-2 (20 µL, LD₉₀ 5,000 PFU). Untreated/infected mice were used as positive controls for infection, while untreated/uninfected mice and infected mice treated with free GRFT (20 µL of 1,000 µg/mL, 20 µg GRFT) served as negative controls. Free GRFT doses were based on previous studies with GRFT gels that were shown to provide *in vivo* protection against HSV-2 infection. Blank PEO fibers were administered as an additional control group in efficacy studies. Mice were monitored daily for 14 days after HSV-2 challenge using an established 4-point scale to monitor the progression of viral infection. Each day, mice were weighed and examined for signs of neurological and epithelial damage. After the two-week period following HSV-2 challenge, mice were euthanized and Kaplan-Meier survival curves were generated. Log-ranked post hoc tests were conducted to assess the statistical significance between groups.

### In Vivo Safety

To assess the safety of fiber administration, mice were similarly subcutaneously injected with 3 mg of Depo-Provera, 5 days prior to fiber administration. Afterward, UV-sterilized blank fibers (5 mg) were intravaginally administered to mice under isoflurane anesthesia using sterile tweezers. Treatment groups included mice administered blank PEO, PVA, or PVP fibers, while control groups included untreated mice, or mice treated with 20 µL free GRFT in PBS (1,000 µg/mL) or 40 µL of Conceptrol gel. An additional sham control was used to mimic fiber administration using tweezers alone. Mice were euthanized 24 or 72 hr after fiber administration and mouse reproductive tracts and vaginal lavages were collected and stored at -80°C following euthanasia (n=3).

The structural integrity of collected reproductive tracts was evaluated using histological analysis. First, tissue samples were washed with PBS, followed by fixation with 4% paraformaldehyde. Samples were then embedded in a paraffin block, and stained with hematoxylin and eosin (H&E). Sample cross-sections were analyzed by a pathologist blinded to the treatment groups.

To determine cytokine levels after blank fiber administration, murine reproductive tracts and vaginal lavages were assessed using a Luminex assay. First, 20 µL of T-Per solution (Thermo Fisher) containing 1% Halt Protease Inhibitor Cocktail (Thermo Fisher) was added per milligram of reproductive tissue. Approximately 20 zirconialsilica beads (BioSpec Productions) were added to each sample, followed by homogenization at 4,500x for 180 s using Precellys 24 homogenizer instrument (Bertin, France). Homogenized samples were cooled on ice for 5 min, and centrifuged at 10,000 x g at 4°C for 5 min. Afterward, sample supernatants were collected, aliquoted, and stored at -80°C for further study. Prior to conducting the Luminex assay, interleukin-1 beta (IL-1β) levels were tested in reproductive tissue samples using specific ELISA Ready-SET-Go! kits (Thermo Fisher). Finally, Luminex assays were used to quantify the cytokine levels in collected mouse tissue and lavages. Cytokines including G-CSF, IFN-gamma, IL-1alpha, IL-1beta, IL-2, IL-6, IP-10, MCP-1, MIP-1alpha, MIP1-beta, MIP-2, and TNF-alpha were selected based on previous studies that examined these markers for intravaginal inflammation and damage.

### Statistical Analysis

Unless otherwise noted, all *in vitro* experiments were conducted in triplicate, with a minimum of 3 replicates per sample. Statistical analysis of samples assessing fiber morphology, fiber characterization, *in vitro* assays, and *in vivo* safety studies was performed by using one-way ANOVA with the Bonferroni post hoc test (p < 0.05). For murine studies assessing viral efficacy, log-ranked post hoc tests were conducted to assess statistical significance as a function of treatment group and survival outcome.

### Fiber Size and Morphology

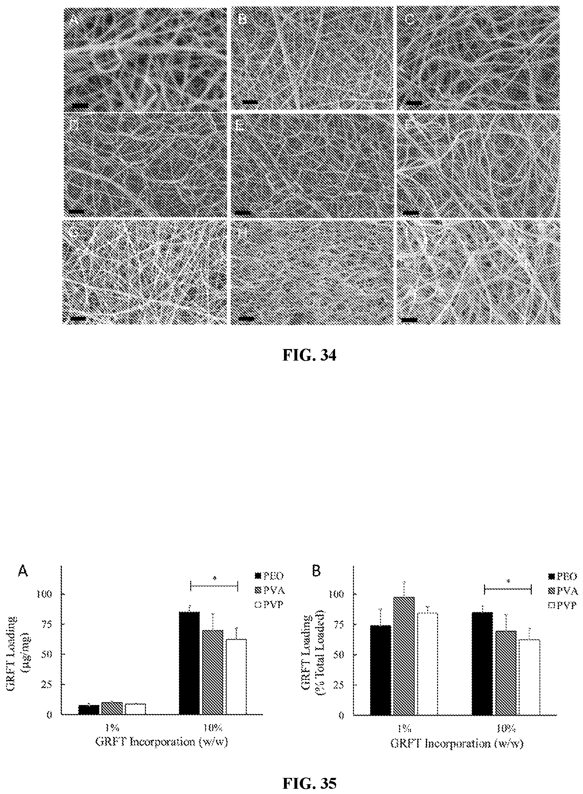

The morphology of blank and GRFT PEO, PVA, and PVP fibers is shown in FIG. 34. All fibers demonstrated well-rounded fiber morphology with average diameters ranging from 220 to 507 nm (Table 4). The addition of 1% GRFT w/w to PEO and PVP fibers resulted in significantly decreased diameters of 239 and 242 nm, while no statistical significance was observed between blank PVA and 1% GRFT PVA fibers that shared similar diameters regardless of GRFT incorporation. The addition of 10% w/w GRFT produced fibers with diameters spanning 243 to 339 nm, demonstrating a statistically significant increase in fiber diameter for 10% w/w GRFT PVA and PVP fibers relative to 1% w/w GRFT PVA and PVP fibers. However, PEO fiber diameters remained unchanged with additional GRFT incorporation. Within similarly loaded GRFT fibers, statistical significance in fiber diameter was observed between the 10% GRFT PEO and PVA fibers, while no statistical significance was observed across the 1% w/w GRFT formulations.

**Table 4. Fiber diameters as a function of polymer formulation and GRFT content. Statistical significance between different polymers with the same GRFT loading is shown as *, while statistical significance between the same polymer type with different GRFT loading is shown as # (p < 0.05)**

| Fiber Formulation | Diameter (nm) |
|---|---|
| Blank PEO | 507 ± 147 |
| Blank PVA | 249 ± 84* |
| Blank PVP | 418 ± 137# |
| PEO 1% GRFT | 239 ± 53# |
| PVA 1% GRFP | 220 ± 59 |
| PVP 1% GRFT | 242 ± 57 |
| PEO 10% GRFT | 243 ± 95* |
| PVA 10% GRFT | 339 ± 99# |
| PVP 10% GRFT | 324 ± 79# |

### Fiber Characterization

GRFT loading was assessed using an ELISA (FIG. 35). For 1% w/w GRFT PEO, PVA, and PVP fibers, GRFT loading ranged from 7.4 to 9.7 µg GRFT/mg fiber, exhibiting no statistical significance between formulations. For 10% w/w GRFT PEO, PVA, and PVP fibers, GRFT loading was 84.8, 69.6, and 62.4 µg GRFT/mg fiber, respectively, with PEO fibers demonstrating statistically higher loading than PVP fibers. Correspondingly, the encapsulation efficiencies for each fiber formulation ranged from 74.0 to 97.2%, and 62.4 to 84.2% for 1 and 10% GRFT fibers, respectively, demonstrating consistently high GRFT loading and suggesting electrospinning compatibility between the polymer and lectin. There were no observable trends between GRFT encapsulation efficiency and fiber diameters.

### In Vitro HIV-1 and HSV-2 Inhibition from GRFT Fibers

The dual-purpose antiviral activity of GRFT fibers was determined using HIV-1 pseudovirus and HSV-2 plaque inhibition assays. For HIV-1 inhibition studies, all fibers demonstrated complete and dose-dependent HIV-1 inhibition (FIG. 36A, 36B). The IC₅₀ values for PEO, PVA, and PVP fibers administered 1 and 24 hr prior to infection ranged from 17.3 ± 7.2 to 26.7 ± 7.7 ng/mL, relative to 24.1 ±15.6 and 22.8 ± 12.2 ng/mL for free GRFT at 1 and 24 hr respectively (Table 5). No statistical significance was observed between the IC₅₀ values of GRFT PEO, PVA, and PVP fibers, suggesting no differences in efficacy based on polymer type or as a function of administration time with respect to cell infection. Moreover, similar inhibition values relative to free GRFT indicate that the electrospinning process maintains the functional activity of GRFT.

Plaque assays were used to assess the ability of GRFT fibers to inhibit HSV-2 infection. Fibers containing a higher concentration of GRFT (10% w/w) were evaluated, due to the increased concentration of GRFT needed to inhibit HSV-2, relative to HIV-1 infection. GRFT PEO, PVA and PVP fibers demonstrated equivalent protection against HSV-2 infection, relative to free GRFT (IC₅₀ 25.5 ± 0.5 µg/mL), with IC₅₀s of 22.0 ± 2.14, 16.6, ± 0.92 and 21.0 ± 2.4 µg/mL (FIG. 36C). No statistical significance in efficacy was observed between GRFT fibers and free GRFT, except for PVA fibers, which showed a lower IC₅₀ value relative to free GRFT (p < 0.05, Table 5). Moreover, administration of all GRFT fiber formulations resulted in decreases in both plaque number and size, relative to untreated/infected controls (FIG. 36D, 36E).

**Table 5. IC50 values from in vitro HIV-1 and HSV-2 infectivity assays. GRFT eluates from rapid-release fibers were assessed against HIV-1 and HSV-2 infections, and compared to free GRFT. Fibers demonstrated comparable activity relative to free GRFT. No statistical significance between groups was observed in HIV-1 inhibition studies as a function of fiber formulation or with respect to administration time. In HSV-2 plaque inhibition assays, GRFT PVA fibers demonstrated lower IC50 values relative to other formulations and free GRFT-treated controls (p < 0.05). The average IC50 values are expressed as the mean ± standard deviation. Statistical significance between GRFT PVA fibers and free GRFT is shown as (*p < 0.05)**

| Fiber Formulation | 1 Hr HIV-1 IC50 (ng/mL) | 24 Hr HIV-1 IC50 (ng/mL) | 1 Hr HSV-2 IC50 (µg/mL) |
|---|---|---|---|
| PEO | 17.9 ± 5.4 | 17.3 ± 7.2 | 22.0 ± 2.1 |
| PVA | 26.7 ± 7.7 | 21.7 ± 14.8 | 16.6 ± 0.9* |
| PVP | 26.6 ± 2.7 | 23.5 ± 14.1 | 21.0 ± 2.4 |
| Free GRFT | 24.1 ± 15.6 | 22.8 ± 12.2 | 25.5 ± 0.5 |

### In Vitro Cytotoxicity

To assess the cytotoxicity of rapid-release fibers, MTT assays were conducted using VK2/, Ect1/, and End1/E6E7 cell lines. All cell lines, incubated with 1 and 10% w/w GRFT fibers, demonstrated greater than 93% viability after 24, 48, and 72 hr fiber administration, relative to untreated cells (FIG. 37). No statistical significance in cell viability was observed as a function of polymer type or GRFT loading.

### In Vivo Efficacy against HSV-2 Infection

The antiviral efficacy of rapid-release GRFT fibers was assessed in a murine model of lethal HSV-2 infection. A single dose of 10% w/w GRFT fibers was intravaginally administered to female BALB/c mice, followed by a single HSV-2 challenge with 5,000 PFU (LD₉₀), 4 hr after fiber insertion (FIG. 38A, 38B). Mice were evaluated daily for progression of HSV-2 infection for 14 d post-infection (FIG. 38C). Mice that were administered GRFT fibers (PEO, PVA, or PVP) exhibited statistically significant decreases in HSV-2 infectivity, with 85, 95, and 100% survivability respectively, relative to untreated/infected controls (5% survivability, p < 0.05). In addition, mice administered GRFT PEO, PVA, or PVP fibers exhibited comparable protection against HSV-2 relative to free GRFT (p > 0.05), while blank PEO fibers imparted protection to 50% of the animals (p < 0.05).

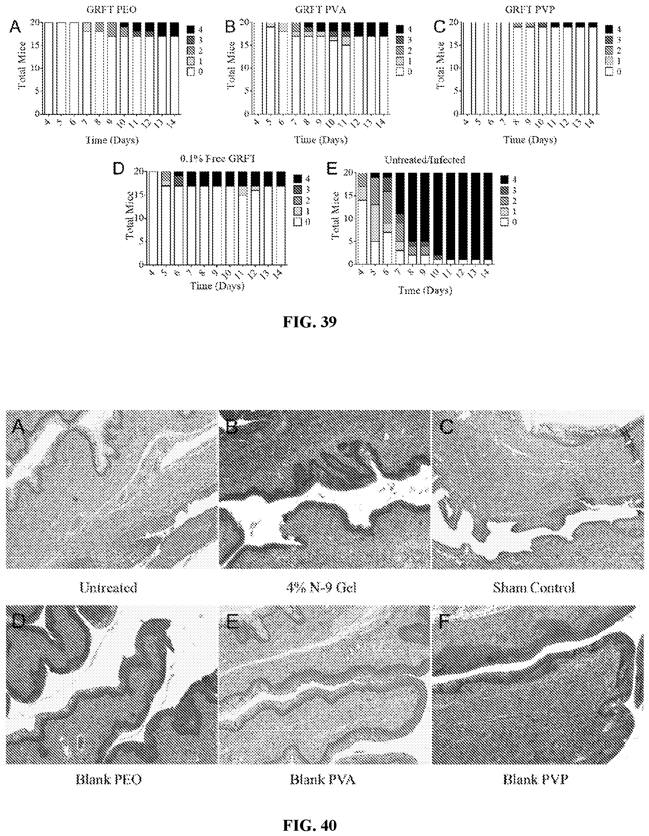

Another important finding from this study was the difference in infection progression between untreated/infected mice and mice treated with GRFT fibers (FIG. 39). The first symptoms of HSV-2 infection in mice typically manifest 4 to 5 days post-infection, during which time mice exhibit symptoms of localized swelling in the vaginal area and decreased hind leg mobility. After 5 to 8 days, -75% of mice from the untreated/infected control group required euthanasia due to the rapid progression and severity of infection. In contrast, all mice administered GRFT fibers (PEO, PVA, or PVP) or free GRFT that exhibited symptoms, showed decreased progression of infection relative to untreated/infected mice over the same duration. The mice that did not survive infection despite pre-treatment with GRFT fibers or free GRFT (representing up to 15% total mice, respectively) exhibited a more gradual progression of infection, requiring euthanasia 7 to 10 d, instead of 5 to 8 d post-infection. The prolonged duration of viral quiescence suggests that GRFT may provide partial protection against infection for the few mice that exhibited overt signs of infection. In comparison, the administration of blank fibers showed no change in progression of HSV-2 infection in mice, relative to untreated/infected controls. Finally, for the few infected mice treated with free GRFT (1 of 20) or GRFT PVA fibers (2 of 20), initial symptoms disappeared near of the end of the study. The decreased levels of infection in combination with the more gradual progression demonstrate the ability of GRFT fibers to protect against a lethal dose of HSV-2 after a single application.

### In Vivo Safety

To assess the *in vivo* safety of rapid-release platforms, fibers were intravaginally administered to mice, and reproductive tissue and vaginal lavages were collected and analyzed 24 and 72 hr following administration.

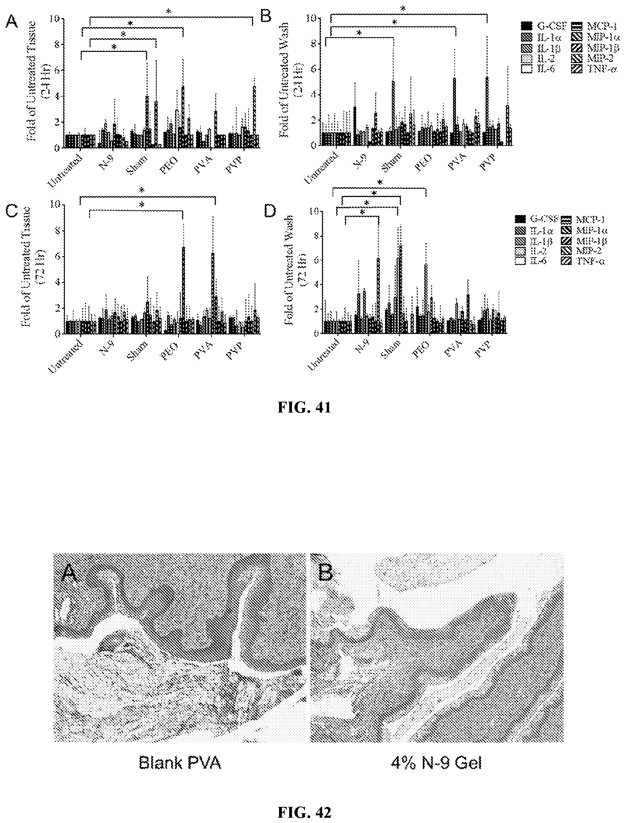

Tissue samples were evaluated for possible edema of muscle, interstitial, and epithelial tissue. In addition, untreated and blank fiber-treated cervical and vaginal epithelia morphologies were compared and assessed for possible keratinization and goblet cell presence. A score ranging from 1 to 4 was used to determine the severity of epithelial changes. FIG. 40 shows images of tissues collected 24 hr after fiber administration. Extracted tissues from untreated controls demonstrated compact squamous and cervical epithelial tissue, with no evidence of edema nor inflammation. Samples from tissues treated with blank PEO, PVA, and PVP fibers showed similar morphology and normality, relative to untreated controls (scores of 1 to 2). Furthermore, there was no increase in lymphocyte accumulation in fiber-treated groups. Overall the tissues from blank fiber-treated mice were comparable to tissues from untreated mice; however, one PVA-treated sample exhibited increased levels of mucin secretion and neutrophil presence (score 3), yet the vaginal and cervical epithelia were intact (FIG. 42A). Results from tissues collected after 72 hr administration were similar to 24 hr samples.

For N-9 treated mice, a slight increase in neutrophil accumulation was observed on the surface of the cervical squamous epithelium, relative to untreated controls, indicating minor topical damage (score range 1 to 2). Murine tissue from one N-9 treated mouse exhibited increased inflammation, due to the presence of peripheral blood mononuclear cells, goblet cell fusions, and epithelial disruption, resulting in a score of 3 (FIG. 42B). As for sham-treated mice, there was a slight increase in neutrophil accumulation after 24 hr in most samples, relative to untreated controls (score range 1 to 2), and one sham-treated replicate was noted for widespread neutrophil accumulation, indicating tissue repair (score 3). These alterations present in both N-9 and sham-treated controls were not observed in 72 hr tissue samples, perhaps indicating transitory damage.

Cytokine expression was assessed from murine FRTs and vaginal lavages 24 and 72 hr after blank fiber administration. Cytokine expression was compared to untreated, N-9 treated, and sham-treated mice based on previously published guidelines, in which a significant level of intravaginal inflammation results in a 2 to 5-fold increase in cytokine expression, relative to untreated controls. FIG. 41 summarizes the results from the cytokine analysis, indicating that blank fibers do not induce pro-inflammatory or immune-regulatory cytokine expression. In fiber-treated tissue and vaginal lavage samples, cytokine levels were found to be comparable to tissue and washes collected from untreated control groups. Cytokine expression was similar in both 24 and 72 hr samples, with 11 of 13 cytokines within a range of 2 to 5-fold expression of untreated controls. A few exceptions were observed using the above criteria: vaginal lavages collected 24 hr after PVA and PVP fiber administration demonstrated a 5-fold increase in IL-1alpha expression, while vaginal tissue collected 24 hr after PEO administration demonstrated elevated levels of MIP1-alpha and PVP MIP-2, relative to untreated controls (p < 0.05). Additionally, vaginal tissue collected 72 hr after PEO fiber administration demonstrated a 6-fold increase in MCP-1 and MIP-1alpha expression, relative to untreated controls (p < 0.05). For sham-treated controls, which simulated the administration method via tweezers only, increased cytokine expression was observed in 24 hr tissue (MIP-2), 24 hr wash (IL-1beta), and 72 hr wash (IL-6, MCP-1), relative to untreated controls. N-9 treated samples demonstrated an increase in MIP-2 expression in 72 hr wash samples only. Both IP-10 and INF-gamma were undetected in vaginal lavage or tissue samples.

An ELISA, used to confirm IL-1beta levels, showed similar results, in that blank fibers induced negligible increases in cytokine expression relative to untreated controls (p > 0.05).

### Example 6-Synergy of Antiretrovirals and Biologic Nanoparticles Against HIV-1 Infection

The following experiments were performed to develop safer and more effective delivery platforms for HIV-1 prevention. Specifically, each active agent was encapsulated in synthetic polymeric NPs (Free Q-GRFT + free ARVs; Q-GRFT NPs + free ARVs; Q-GRFT NPs + ARV NPs) and the efficacy of each was assessed. The release of agents can be prolonged by using NP-EF composites.

### Nanoparticle Fabrication

Dichloromethane (DCM) was used as the organic phase, and polyvinyl alcohol (PVA) was used as the emulsifier or aqueous phase. An active agent (see below) was added to the DCM/PLGA, which was subsequently added to the PVA. The resulting emulsion was added to a larger aqueous phase and stirred until the DCM was evaporated. The resulting NPs were centrifuged, washed and lyophilized.

### Nanopore Characterization

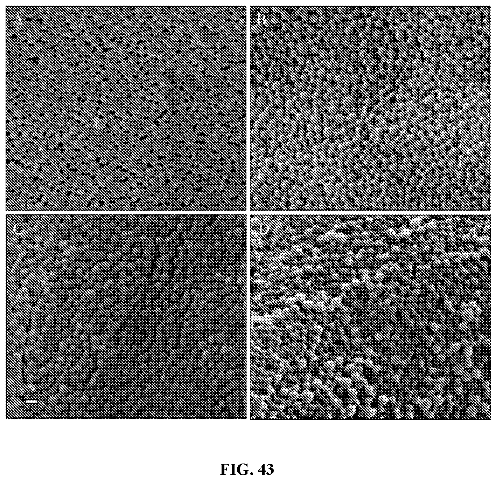

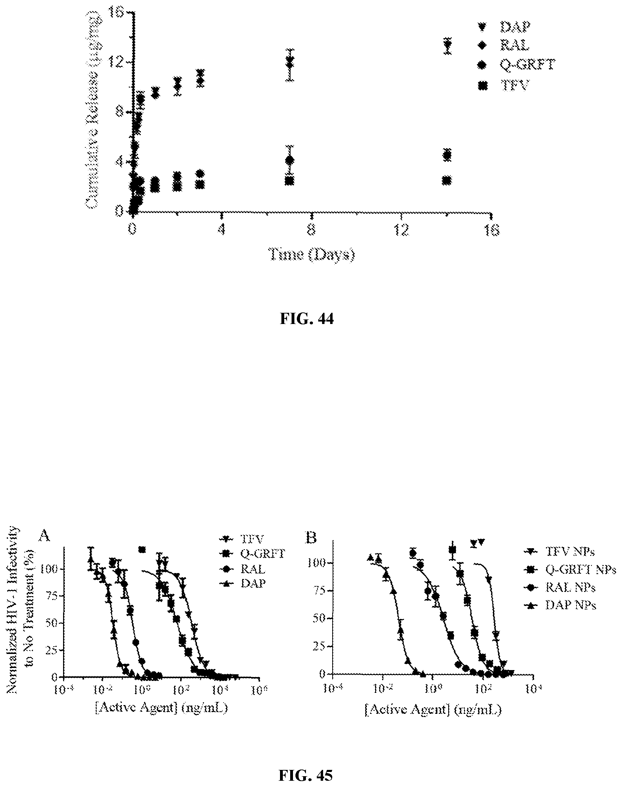

PLGA NPs encapsulating an active agent (i.e., 10% Q-GRFT, TFV, DAP or RAL) were synthesized with high yield, and their morphology was imaged using scanning electron microscope (SEM). See FIG. 43. In addition, the release time of the various active agents from the PLGA NPs also was evaluated. See FIG. 44.

### Efficacy of Active Agents Against HIV-1

An HIV inhibition assay was applied using the various active agents. See, for example, Piehler et al., 2011, "LabKey Server NAb: A tool for analyzing, visualizing and sharing results from neutralizing antibody assays," BMC Immunology, 12:33.

Briefly, TZM-bl cells were incubated with each active agent for 1 hr, and modified HIV-1 was added to the media. After 48 hrs, luciferase was added, and, in a luciferase assay, any infected cells exhibited luminescence. Since infection was proportional to the amount of luminescence, the half maximal inhibitory concentration (IC₅₀) was calculated. The IC50 of free active agent (FIG. 45A) and encapsulated active agent (FIG. 45B) is shown.

### Co-Administering Active Agents Against HIV

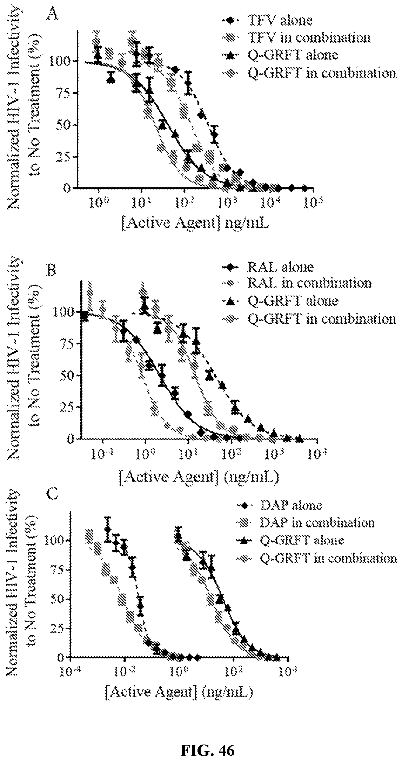

Free Q-GRFT was co-administered with free ARVs (TFV + Q-GRFT (FIG. 46A); RAL + Q-GRFT (FIG. 46B); DAP + Q-GRFT (FIG. 46C) and IC₅₀ values were determined. Results determined that the IC50 of free Q-GRFT was up to 6-fold lower when administered in combination with the free ARVs, and that the IC50 of the free ARVs was up to 5.5-fold lower when administered in combination with free Q-GRFT.

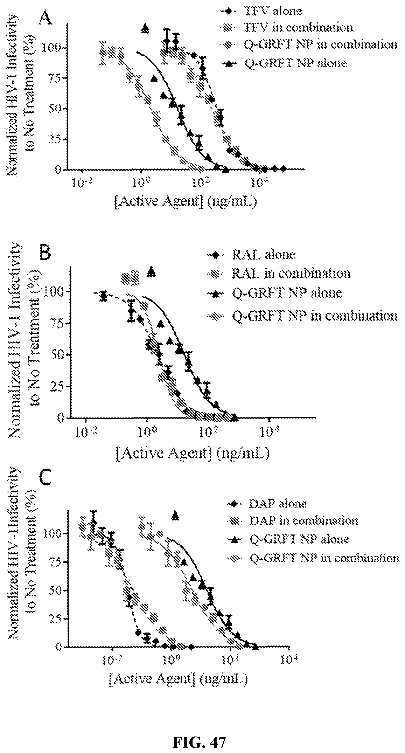

Next, Q-GRFT NPs were co-administered with free ARVs (TFV + Q-GRFT NP (FIG. 47A); RAL + Q-GRFT NP (FIG. 47B); DAP + Q-GRFT NP (FIG. 47C) and IC50 values were determined. Results determined that the IC50 of Q-GRFT NPs was up to 12-fold lower in combination with free ARVs, and that the IC50 of free ARVs was up to 1.5-fold lower in combination with Q-GRFT NPs.

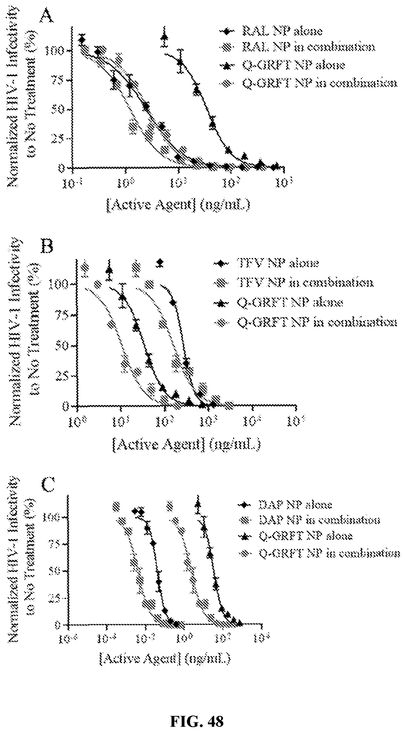

In addition, Q-GRFT NPs were co-administered with encapsulated ARVs (TFV NP + Q-GRFT NP (FIG. 48A); RAL NP + Q-GRFT NP (FIG. 48B); DAP NP + Q-GRFT NP (FIG. 48C)) and IC50 values were determined. The IC50 of Q-GRFT NPs was up to 15-fold lower when administered in combination with encapsulated ARVs, and the IC50 of encapsulated ARVs was up to 7-fold lower when administered in combination with Q-GRFT NPs.

### In Vitro Cytotoxicity of NPs

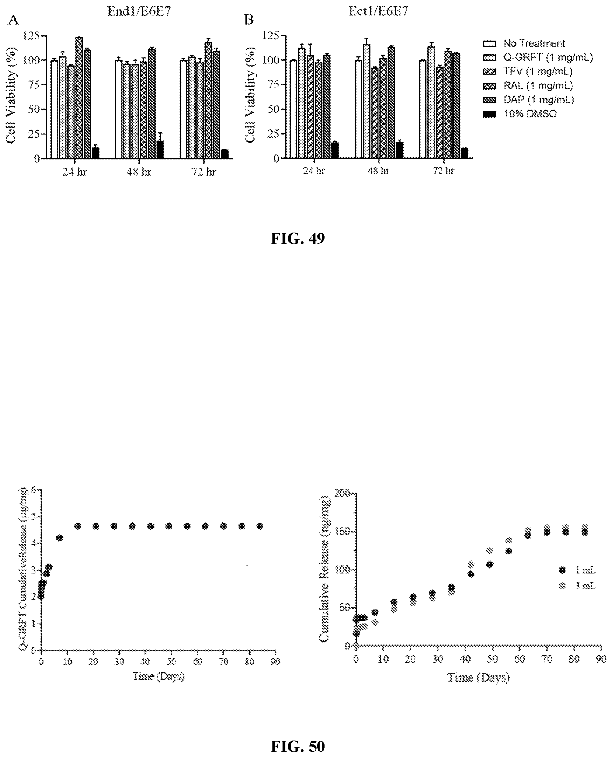

The MTT assay was used to determine the *in vitro* cytotoxicity of the active agent NPs. FIG. 49A shows the MTT cytotoxic assay with the various active agent NPs in End1/E6E7 cells, while FIG. 49B shows the MTT cytotoxic assay with the various active agent NPs in Ect1/E6E7 cells.

### Release of Active Agents from Multilayer NP-EF Composites

NPs were suspended in an inner layer polymer solution and electrospun. In this type of structure, the hydrophilic inner layer acts as a reservoir for NPs and the hydrophobic outer layer minimizes the active release. The release of Q-GRFT was evaluated from either NPs (FIG. 50A) or NP-EF composites (FIG. 50B). NP-EF performed extremely well, and, therefore, NP-EF composites can be used to improve the efficacy as well as the release of active agents. Surprisingly, the NP-EF delivery platform demonstrated sustained-release for up to 90 days.

Therefore, incorporating NPs encapsulating different agents in such a platform may result in prolonged release of active agents as well as increasing the efficacy due to, for example, synergistic interactions.

### Conclusions

Experiments herein have demonstrated that TFV, RAL, and DAP have synergistic interactions with Q-GRFT against HIV-1 in free and encapsulated forms. Coadministration of encapsulated agents with different mechanisms of action may increase the prophylactic effect and decrease the dose needed to attain efficacy against HIV-1 infections. The NP-EF composites provided prolonged release of active agents.

## Claims

1. An antimicrobial composition comprising: electrospun fibers comprising a probiotic embedded therein, wherein the probiotic is a *Lactobacillus* spp. selected from the group consisting of *L. acidophilus*, *L. crispatus*, *L. rhamnosus*, *L. reuteri*, *L. jensenii*, and *L. gasseri*, wherein the antimicrobial composition further comprises a prebiotic, and wherein the prebiotic is selected from the group consisting of fructans (e.g., fructooligosaccharides (FOS), inulins), galactans (galactooligosaccharides (GOS)), pectin, beta-glucans, and xylooligosaccharides.

2. The antimicrobial composition of claim 1, wherein the electrospun fiber material is biodegradable electrospun fiber material.

3. The antimicrobial composition of claim 2, wherein the biodegradable fiber material is selected from the group consisting of PLGA, mPEG-PLGA, PVA, PEO, PVP, and combinations thereof.

4. The antimicrobial composition of claim 1, wherein the electrospun fiber material is non-biodegradable electrospun fiber material.

5. The antimicrobial composition of claim 4, wherein the non-biodegradable electrospun fiber material is polycaprolactone (PCL).

6. A method of making an antimicrobial composition, comprising:
electrospinning fiber material and a probiotic into electrospun fibers comprising the probiotic embedded therein,
thereby making an antimicrobial composition, wherein the probiotic is a *Lactobacillus* spp. selected from the group consisting of *L. crispatus*, *L. rhamnosus*, *L. reuteri*, *L. jensenii*, and *L. gasseri*, and wherein the antimicrobial composition further comprises a prebiotic, wherein the prebiotic is selected from the group consisting of fructans (e.g., fructooligosaccharides (FOS), inulins), galactans (galactooligosaccharides (GOS)), pectin, beta-glucans, and xylooligosaccharides.

7. The antimicrobial composition of any of claims 1 to 5 for use in a method of inhibiting the growth and/or reducing the amount of BV-associated bacteria (BVAB) in a BVAB infection in a female, comprising:
implanting the antimicrobial composition of any of claims 1 to 5 into the reproductive tract of the female.

8. The antimicrobial composition for use of claim 7, wherein the implanting step changes the pH of the female reproductive tract.

9. The antimicrobial composition for use of claim 7, wherein the implanting step results in the production or increased production of lactic acid.

10. The antimicrobial composition for use of claim 7, wherein the implanting step changes the pH of the female reproductive track and results in the production or increased production of lactic acid.

11. The antimicrobial composition for use of any of claims 7-10, wherein the BVAB infection comprises the presence of at least one bacteria.

12. The antimicrobial composition for use of claim 11, wherein the bacteria is selected from the group consisting of *Gardnerella vaginalis*, *Atopobium vaginae*, and *Neisseria gonorrhoeae.*

13. The antimicrobial composition for use of any of claims 7-12, wherein the BVAB infection comprises the presence of at least one protozoa.

14. The antimicrobial composition for use of claim 13, wherein the protozoa is T. vaginalis.

15. The antimicrobial composition for use of any of claims 7-14, wherein the implanting step provides a sustained release of the probiotic into the reproductive tract of the female.

## Patentansprüche

1. Antimikrobielle Zusammensetzung, umfassend: elektrogesponnene Fasern, die ein darin eingebettetes Probiotikum umfassen, wobei es sich bei dem Probiotikum um eine *Lactobacillus* spp., ausgewählt aus der Gruppe, bestehend aus *L. acidophilus*, *L. crispatus*, *L. rhamnosus*, *L. reuteri*, *L. jensenii* und *L. gasseri*, handelt, wobei die antimikrobielle Zusammensetzung ferner ein Präbiotikum umfasst, und wobei das Präbiotikum ausgewählt ist aus der Gruppe, bestehend aus Fructanen (z. B. Fructooligosacchariden (FOS), Inulinen), Galactanen (Galactooligosacchariden (GOS)), Pectin, Beta-Glucanen und Xylooligosacchariden.

2. Antimikrobielle Zusammensetzung nach Anspruch 1, wobei es sich bei dem elektrogesponnenen Fasermaterial um biologisch abbaubares elektrogesponnenes Fasermaterial handelt.

3. Antimikrobielle Zusammensetzung nach Anspruch 2, wobei das biologisch abbaubare Fasermaterial ausgewählt ist aus der Gruppe, bestehend aus PLGA, mPEG-PLGA, PVA, PEO, PVP und Kombinationen davon.

4. Antimikrobielle Zusammensetzung nach Anspruch 1, wobei es sich bei dem elektrogesponnenen Fasermaterial um nicht biologisch abbaubares elektrogesponnenes Fasermaterial handelt.

5. Antimikrobielle Zusammensetzung nach Anspruch 4, wobei es sich bei dem nicht biologisch abbaubaren elektrogesponnenen Fasermaterial um Polycaprolacton (PCL) handelt.

6. Verfahren zur Herstellung einer antimikrobiellen Zusammensetzung, umfassend:
Elektrospinnen von Fasermaterial und einem Probiotikum zu elektrogesponnenen Fasern, die das darin eingebettete Probiotikum umfassen,
wodurch eine antimikrobielle Zusammensetzung hergestellt wird, wobei es sich bei dem Probiotikum um eine *Lactobacillus* spp., ausgewählt aus der Gruppe, bestehend aus *L. crispatus*, *L. rhamnosus*, *L. reuteri*, *L. jensenii* und *L. gasseri*, handelt, und wobei die antimikrobielle Zusammensetzung ferner ein Präbiotikum umfasst, wobei das Präbiotikum ausgewählt ist aus der Gruppe, bestehend aus Fructanen (z. B. Fructooligosacchariden (FOS), Inulinen), Galactanen (Galactooligosacchariden (GOS)), Pectin, Beta-Glucanen und Xylooligosacchariden.

7. Antimikrobielle Zusammensetzung nach einem der Ansprüche 1 bis 5 zur Verwendung in einem Verfahren zur Hemmung des Wachstums und/oder Verringerung der Menge von BV-assoziierten Bakterien (BVAB) bei einer BVAB-Infektion in einer Frau, umfassend:
Implantieren der antimikrobiellen Zusammensetzung nach einem der Ansprüche 1 bis 5 in den Reproduktionstrakt der Frau.

8. Antimikrobielle Zusammensetzung zur Verwendung nach Anspruch 7, wobei der Schritt des Implantierens den pH-Wert des weiblichen Reproduktionstrakts verändert.

9. Antimikrobielle Zusammensetzung zur Verwendung nach Anspruch 7, wobei der Schritt des Implantierens zur Produktion oder erhöhten Produktion von Milchsäure führt.

10. Antimikrobielle Zusammensetzung zur Verwendung nach Anspruch 7, wobei der Schritt des Implantierens den pH-Wert des weiblichen Reproduktionstrakts verändert und zur Produktion oder erhöhten Produktion von Milchsäure führt.

11. Antimikrobielle Zusammensetzung zur Verwendung nach einem der Ansprüche 7-10, wobei die BVAB-Infektion das Vorhandensein von wenigstens einem Bakterium umfasst.

12. Antimikrobielle Zusammensetzung zur Verwendung nach Anspruch 11, wobei das Bakterium ausgewählt ist aus der Gruppe, bestehend aus *Gardnerella vaginalis*, *Atopobium vaginae* und *Neisseria gonorrhoeae.*

13. Antimikrobielle Zusammensetzung zur Verwendung nach einem der Ansprüche 7-12, wobei die BVAB-Infektion das Vorhandensein von wenigstens einem Protozoon umfasst.

14. Antimikrobielle Zusammensetzung zur Verwendung nach Anspruch 13, wobei es sich bei dem Protozoon um T. vaginalis handelt.

15. Antimikrobielle Zusammensetzung zur Verwendung nach einem der Ansprüche 7-14, wobei der Schritt des Implantierens eine anhaltende Freisetzung des Probiotikums in den Reproduktionstrakt der Frau bereitstellt.

## Revendications

1. Composition antimicrobienne comprenant : des fibres électrofilées comprenant un probiotique incorporé dans celles-ci, le probiotique étant un *Lactobacillus* spp. choisi dans le groupe constitué par *L. acidophilus*, *L. crispatus*, *L. rhamnosus*, *L. reuteri*, *L. jensenii* et *L. gasseri*, la composition antimicrobienne comprenant en outre un prébiotique, et le prébiotique étant choisi dans le groupe constitué par des fructanes (par exemple, fructooligosaccharides (FOS), inulines), des galactanes (galactooligosaccharides (GOS)), une pectine, des bêta-glucanes et des xylooligosaccharides.

2. Composition antimicrobienne selon la revendication 1, le matériau de fibres électrofilées étant un matériau de fibres électrofilées biodégradables.

3. Composition antimicrobienne selon la revendication 2, le matériau de fibres biodégradables étant choisi dans le groupe constitué par PLGA, mPEG-PLGA, PVA, PEO, PVP, et des combinaisons correspondantes.

4. Composition antimicrobienne selon la revendication 1, le matériau de fibres électrofilées étant un matériau de fibres électrofilées non biodégradables.

5. Composition antimicrobienne selon la revendication 4, le matériau de fibres électrofilées non biodégradables étant une polycaprolactone (PCL).

6. Procédé de préparation d'une composition antimicrobienne, comprenant :
un matériau de fibres d'électrofilage et un probiotique dans des fibres électrofilées comprenant le probiotique incorporé dans celles-ci,
préparant ainsi une composition antimicrobienne, le probiotique étant un *Lactobacillus* spp. choisi dans le groupe constitué par *L. crispatus*, *L. rhamnosus*, *L. reuteri*, *L. jensenii* et *L. gasseri*, et la composition antimicrobienne comprenant en outre un prébiotique, le prébiotique étant choisi dans le groupe constitué par des fructanes (par exemple, fructooligosaccharides (FOS), inulines), des galactanes (galactooligosaccharides (GOS)), une pectine, des bêta-glucanes et des xylooligosaccharides.

7. Composition antimicrobienne selon l'une quelconque des revendications 1 à 5, pour une utilisation dans un procédé d'inhibition de la croissance et/ou de réduction de la quantité de bactéries associées à BV (BVAB) dans une infection par BVAB chez une femme, comprenant :
l'implantation de la composition antimicrobienne selon l'une quelconque des revendications 1 à 5 dans l'appareil génital de la femme.

8. Composition antimicrobienne pour une utilisation selon la revendication 7, l'étape d'implantation modifiant le pH de l'appareil génital de la femme.

9. Composition antimicrobienne pour une utilisation selon la revendication 7, l'étape d'implantation entraînant la production ou la production augmentée d'acide lactique.

10. Composition antimicrobienne pour une utilisation selon la revendication 7, l'étape d'implantation modifiant le pH de l'appareil génital de la femme et entraînant la production ou la production augmentée d'acide lactique.

11. Composition antimicrobienne pour une utilisation selon l'une quelconque des revendications 7 à 10, l'infection par BVAB comprenant la présence d'au moins une bactérie.

12. Composition antimicrobienne pour une utilisation selon la revendication 11, la bactérie étant choisie dans le groupe constitué par *Gardnerella vaginalis*, *Atopobium vaginae*, et *Neisseria gonorrhoeae.*

13. Composition antimicrobienne pour une utilisation selon l'une quelconque des revendications 7 à 12, l'infection par BVAB comprenant la présence d'au moins un protozoaire.

14. Composition antimicrobienne pour une utilisation selon la revendication 13, le protozoaire étant T. vaginalis.

15. Composition antimicrobienne pour une utilisation selon l'une quelconque des revendications 7 à 14, l'étape d'implantation fournissant une libération prolongée du probiotique dans l'appareil génital de la femme.
